(19) **Europäisches Patentamt**
European Patent Office
Office européen des brevets

(11) **EP 4 296 349 A2**

(12) ## EUROPÄISCHE PATENTANMELDUNG

(43) Veröffentlichungstag:
**27.12.2023 Patentblatt 2023/52**

(21) Anmeldenummer: **23180517.7**

(22) Anmeldetag: **21.06.2023**

(51) Internationale Patentklassifikation (IPC):
*C12M 1/34* (2006.01)    *G01N 21/80* (2006.01)
*C12M 1/12* (2006.01)    *C12M 1/00* (2006.01)

(52) Gemeinsame Patentklassifikation (CPC):
**C12M 41/26; C12M 25/04; C12M 25/14;
C12M 29/00; C12M 41/34; G01N 21/80**

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**KH MA MD TN**

(30) Priorität: **22.06.2022 DE 102022115584**

(71) Anmelder: **Karlsruher Institut für Technologie
76131 Karlsruhe (DE)**

(72) Erfinder:
• **Grün, Christoph
  Landau (DE)**
• **Gottwald, Eric
  Karlsruhe (DE)**

(74) Vertreter: **Gille Hrabal Partnerschaftsgesellschaft
mbB
Patentanwälte
Brucknerstraße 20
40593 Düsseldorf (DE)**

(54) **DREIDIMENSIONAL STRUKTURIERTE SENSORFOLIEN**

(57) Die Erfindung betrifft dreidimensionale Strukturen zur Aufnahme und Kultur von Zellen, die aus funktionalisierten Sensorfolien gebildet sind und eine Analytmessung in der Zellkultur ermöglichen, sowie Verfahren zu deren Herstellung, Verfahren zur Messung von Analyten oder Analytgradienten in einer Zellkultur mittels der erfindungsgemäßen dreidimensionalen Strukturen, sowie Vorrichtungen umfassend die erfindungsgemäßen dreidimensionalen Strukturen und geeignete Detektions- und Messdatenverarbeitungseinheiten.

EP 4 296 349 A2

**Beschreibung**

**EINLEITUNG**

Die vorliegende Erfindung betrifft dreidimensionale Strukturen zur Aufnahme und Kultur von Zellen, die aus funktionalisierten Sensorfolien gebildet sind und eine Analytmessung in der Zellkultur ermöglichen. Die Erfindung umfasst außerdem Verfahren zur Herstellung solcher Analyt-sensitiver dreidimensionaler Strukturen, Verfahren zur Messung von Analyten oder Analytgradienten in einer Zellkultur mittels der erfindungsgemäßen dreidimensionalen Strukturen, die Verwendung der erfindungsgemäßen dreidimensionalen Strukturen zur Zellkultivierung und Analytmessung in der Zellkultur, sowie Vorrichtungen umfassend die erfindungsgemäßen dreidimensionalen Strukturen und geeignete Detektions- und Messdatenverarbeitungseinheiten.

**HINTERGRUND**

In Zellkulturen spielen gewebetypische Analytkonzentrationen, wie z.B. die Sauerstoff-, $CO_2$- und Glukosekonzentration oder der pH-Wert eine wichtige Rolle. Informationen zum Sauerstoffgradienten bzw. die exakte Messung der Sauerstoffkonzentration in der Mikroumgebung dreidimensionaler Zellkulturen sind notwendig, um bspw. Aussagen über die Versorgung der Zellen treffen zu können und zu beurteilen, ob die Kulturbedingungen der physiologischen Situation entsprechen.

In der Regel werden Zellkulturen unter Umgebungsatmosphäre, also mit einer Konzentration von 21 % Sauerstoff kultiviert, die in keinem Gewebe erreicht wird und somit zu einer Sauerstoffüberversorgung (Hyperoxie) führt, was Stressbedingungen in der Zellkultur mit unphysiologischen Antworten auf äußere Reize, wie z.B. auf Chemikalien, führt. Um gewebetypische Sauerstoffkonzentrationen in Zellkulturen einstellen zu können, sind entsprechende Analyseverfahren notwendig. Sauerstoff kann hierzu bspw. polarographisch bestimmt werden, was jedoch wiederum mit Sauerstoffverbrauch verbunden ist und nicht für solche Anwendungen geeignet ist. Für Zellkulturen ebenso wenig geeignet ist die Winkler-Methode, bei der Sauerstoff durch Zugabe von Manganchlorid und Kaliumiodid und dem daraus entstehenden braunen Niederschlag aus Manganoxidhydroxid ermittelt wird.

Ein alternativer Ansatz der Sauerstoffmessung beruht auf der dynamischen Fluoreszenzlöschung. Hierbei wird ein Fluorophor durch Bestrahlen mit Licht einer bestimmten Wellenlänge angeregt, welches daraufhin das Fluoreszenzemissionslicht entweder abgibt oder strahlungsfrei auf ein Sauerstoffmolekül überträgt. Dadurch sinkt die Emission, wodurch die Sauerstoffkonzentration bestimmt werden kann. Solche Systeme sind weit verbreitet und werden von einigen Firmen kommerziell vertrieben. Hierbei sind die Fluorophore entweder auf eine flexible Polymerträgerfolie aufgebracht oder in Form von Hydrogelen oder in Nanopartikeln immobilisiert. Als Fluorophore werden hierfür häufig Platin-II- bzw. Palladium-II-Porphyrine oder Ruthenium-II-Komplexe verwendet [Wang, X.D. and O.S. Wolfbeis: Optical methods for sensing and imaging oxygen: materials, spectroscopies and applications; Chem Soc Rev, 2014. 43(10): p. 3666-761].

Für eine zweidimensionale Kultivierung von Zellen sind solche planaren Foliensysteme ausreichend. Eine zweidimensionale Anordnung von Zellen kommt jedoch, außer an der Innenwand von Blutgefäßen und in der Lunge, im menschlichen Körper nicht vor. Stattdessen erfahren (*in vivo*) Gewebe Sauerstoffgradienten, die mit der Dicke der Gewebe zunehmen bzw. durch Blutgefäße abgefedert werden. Die Bestimmung von Sauerstoffkonzentrationen in unmittelbarer Mikroumgebung von 3D-Zellkulturen, wie bspw. in Zellaggregaten oder Sphäroiden, sowie die Bestimmung von Sauerstoffgradienten, ist mit oben beschriebenen Verfahren bislang nicht möglich. Dies ist jedoch enorm wichtig für die Etablierung organotypischer Zell- und Gewebekulturen, damit eine verbesserte Übertragbarkeit solcher Versuche auf die menschliche Situation gewährleistet ist. Insbesondere für Mikrokavitätenarrays zur Generierung von 3D-Zellkulturen ist bisher kein System oder Verfahren bekannt, mit dem sich die Sauerstoffkonzentrationen oder gar Sauerstoffgradienten ortsaufgelöst messen lassen.

Auch für andere Zellkultur-relevante Analyten wie $CO_2$, Glukose oder den pH-Wert, sowie deren Gradienten, sind bisher keine entsprechend geeigneten Systeme oder Verfahren bekannt.

**STAND DER TECHNIK**

In der Vergangenheit wurden bereits folienbasierte Bioreaktoren oder mikrostrukturierte Formkörper aus thermoverformbaren Folien beschrieben. So beschreibt die DE 10 2007 007 718 A1 einen Bioreaktor, der aus einem warmumformbaren Folienmaterial besteht, sowie dessen Herstellungsprozess und dessen mögliche Verwendung zur Kultivierung von Zellmaterial. Die darin beschriebenen Bioreaktoren werden lediglich für die Verwendung zur Zellkultivierung beschrieben. Eine Ausgestaltung in Form von Mikrokavitäten wird nicht erwähnt.

Die DE 10 2009 044 115 A1 beschreibt mikrostrukturierte Formkörper mit perforierten Teilen, welche in Form perforierter Kavitäten ausgebildet sein können, und die auf einem zwei-lagigen Foliensystem basieren, sowie Verfahren zur Herstellung eines solchen mikrostrukturierten Formkörpers. Auch die hierin beschriebenen Mikrostrukturen sind für die Verwendung zur Zellkultivierung vorgesehen.

Die hierin beschriebenen Bioreaktoren oder Formkörper eignen sich zur Zellkultivierung, weisen bisher aber keine Möglichkeit auf, non-invasiv wichtige zu erfassende Messparameter, wie die Sauerstoffkonzentration in der Zellumgebung, zu bestimmen. Eine Verwendung derartiger Bioreaktoren oder Formkörper als Biosensor wird folglich nicht beschrieben. Auch wird eine mögliche Funktionalisierung der darin eingesetzten Folien nicht erwähnt.

Die DE10 2004 035 267 B3 beschreibt folienbasierte Formkörper, in die mindestens eine Hohlstruktur eingebracht ist, wobei in die Folien und die mindestens eine Hohlstruktur (Kavität) eine Vielzahl von Poren eingebracht werden. Diese Formkörper können durch ein darin beschriebenes Mikrothermoformverfahren hergestellt werden. Auch die hierin beschriebenen mikrostrukturierten porösen Formkörper werden primär für die Züchtung und Kultivierung von Zellen vorgesehen. Hierin wird außerdem allgemein die Möglichkeit einer Funktionalisierung der Mikrokavitäten durch Sammeln von Mikro- oder Nanopartikeln und Oberflächenfunktionalisierung dieser Partikel durch Perfusion von Reaktionsmedien erwähnt. Außerdem wird erwähnt, dass die Immobilisierung von Enzymen auf den Oberflächen der Kavitäten den Aufbau eines Biosensors ermöglicht.

Wie bereits erwähnt, sind Methoden für die Bestimmung von Sauerstoffkonzentrationen grundsätzlich bekannt, wobei sich die oben beschriebenen Bestimmungsmethoden aus den vorstehend genannten Gründen meist nicht im Zellkulturbereich anwenden lassen.

Dagegen lassen sich Fluoreszenz-optische Methoden auf Grundlage der dynamischen Fluoreszenzlöschung, wie nachfolgend näher beschrieben, gut auch im Bereich von Zellkulturen einsetzen. Eine bekannte Methode basiert auf Fluorophoren, die kovalent an Nanopartikel gebunden werden [Nichols, A.J., et al.: Clickassembled, oxygen-sensing nanoconjugates for depthresolved, near-infrared imaging in a 3D cancer model. Angew Chem Int Ed Engl, 2014. 53(14): p. 3671-4]. Der Einsatz von Nanopartikeln ist jedoch insofern nachteilig, als diese von den Zellen aufgenommen werden und damit einen unerwünschten (artifiziellen) Einfluss auf die Zellen haben. Die Verwendung von Nanopartikel-gebundenen Fluorophoren ist somit ebenfalls für viele Fragestellung nicht geeignet. Zudem sind sowohl Hydrogel- als auch Nanopartikel-basierte Systeme in ihrer Herstellung und Lagerung aufwändig, die Haltbarkeit ist mitunter begrenzt und hängt von den Lagerbedingungen ab.

Durch die Verwendung von Lichtwellenleitern in Verbindung mit optischen Sensoren können ebenfalls Sauerstoffkonzentrationen bestimmt werden. Hierfür sind Mikrosensoren bereits kommerziell erhältlich. Solche Mikrosensoren können über Aktuatoren bewegt werden, wodurch auch Messungen entlang der z-Achse möglich sind, was wiederum die Messung von Sauerstoffgradienten ermöglicht. [Eggert, S., et al.: Automated 3D Microphysiometry Facilitates High-Content and Highly Reproducible Oxygen Measurements within 3D Cell Culture Models; ACS Sens, 2021. 6(3): p. 1248-1260]. Ein großer Nachteil hierbei ist der vergleichsweise hohe apparative Aufwand, um mit entsprechender Genauigkeit reproduzierbar messen zu können. Dadurch, dass die Mikrosensoren mechanisch bewegt werden müssen, ist dadurch auch die Datengenerierung verlangsamt. Durch den hohen apparativen Aufwand und die langen Messzeiten eignen sich solche Systeme deshalb nicht für Hochdurchsatzverfahren. Zudem sind solche Mikrosensoren deutlich zu groß für die Messung in Mikrokavitäten bzw. in der unmittelbaren Mikroumgebung von 3D-Zellkulturen.

Eine weitere, weit verbreitete Methode basiert auf der Verwendung von Folien, auf die entsprechende Fluorophore aufgebracht bzw. darauf immobilisiert werden [Babilas, P., et al.: In vivo phosphorescence imaging of pO2 using planar oxygen sensors: Microcirculation, 2005. 12(6): p. 477-87 and Kellner, K., et al.: Determination of oxygen gradients in engineered tissue using a fluorescent sensor; Biotechnol Bioeng, 2002. 80(1): p. 73-83]. Bei den darin eingesetzten Folien handelt es sich um planare Folien, die für Anwendungen im zweidimensionalen Bereich geeignet sind.

Für die Messung von Sauerstoffkonzentrationen über optische Verfahren in dreidimensionalen Zellkulturen bietet sich zudem an, Fluorophore in eine Matrix, bspw. ein Hydrogel, einzubinden [Valimaki, H., et al.: Covalent immobilization of luminescent oxygen indicators reduces cytotoxicity; Biomed Microdevices, 2020. 22(2): p. 41]. Ein weiteres Beispiel eines Biosensors, basierend auf Fluorophoren, die in einer Hydrogelmatrix dispergiert sind, wurde für die Anwendung von Fluoreszenz-optischen Methoden zur Untersuchung von Sauerstoffgradienten *in vivo* an chronischen Wunden beschrieben [Schreml et al.; Luminescent Dual Sensors Reveal Extracellular pH-Gradients and Hypoxia on Chronic Wounds That Disrupt Epidermal Repair. Theranostics, 2014. 4(7): p. 721-735]. Die Verwendung von Sauerstoffsensitiven Hydrogelen ist für bestimmte Fragestellungen sinnvoll, jedoch ist man hier auf die Verwendung von Hydrogelen festgelegt. In zahlreichen Anwendungsfällen werden 3D-Zellkulturen in Form von Sphäroiden und damit Hydrogel-frei generiert, sodass dann derartige Methoden nicht verwendet werden können.

Sauerstoff-sensitive Folien auf Basis Fluorophor-beschichteter bzw. -dotierter Sensorfolien zur Sauerstoffbestimmung in zweidimensionalen Zellkulturen sind bereits grundsätzlich bekannt und kommerziell erhältlich. Sauerstoff-sensitive Folien sind im Vergleich zu Hydrogelen und Nanopartikeln unproblematischer in ihrer Handhabung und eignen sich prinzipiell auch für Rolle-zu-Rolle-Herstellungsverfahren.

In der Publikation von Peniche Silva et al.: A New Non-invasive Technique for Measuring 3D-Oxygen Gradients in Wells During Mammalian Cell Culture; Original Research, 17. Juni 2020, doi: 10.3389/fbioe.2020.00595 wird die Verwendung derartiger kommerzieller Sensorfolien zur Messung von Sauerstoffgradienten in Zellkulturen beschrieben. Darin wird die Sensorfolie auf eine 3D-gedruckte Rampe mittels Haftmittel aufgebracht und kann als Insert in Zellkulturmultiwellplatten eingebracht werden. Entlang der Rampe können mit diesem System ebenfalls Sauerstoffgradienten entlang der z-Achse gemessen werden.

Ein solches Modell ist auch Gegenstand der WO201908461A1. Diese beschreibt eine Vorrichtung, ein Verfahren und ein Detektionselement zur Gewinnung von zumindest 2D-Analytinformationen. Darin wird eine Kamera mit einem 2-dimensionalen Sensorchip und einer Beleuchtungseinrichtung zur Erzeugung eines Beleuchtungslichts versehen. Mindestens ein Detektionselement ist mit einer Sensorfolie versehen. Über das Detektionselement wird die 2-dimensionale Sensorfolie beleuchtet und eine Kamera bildet das Licht von der Sensorfolie auf einen Sensorchip ab. Es wird beschrieben, dass die vorgesehene Sensorfolie in der beschriebenen Vorrichtung in der Lage ist, mit Mikrometerauflösung Analytunterschiede zu erfassen und in ein nicht-homogenes flächiges Lichtsignal zu übersetzen, wobei die "Nicht-Homogenität" 1:1 einen anwesenden Analytgradienten oder eine Analytverteilung widerspiegeln soll.

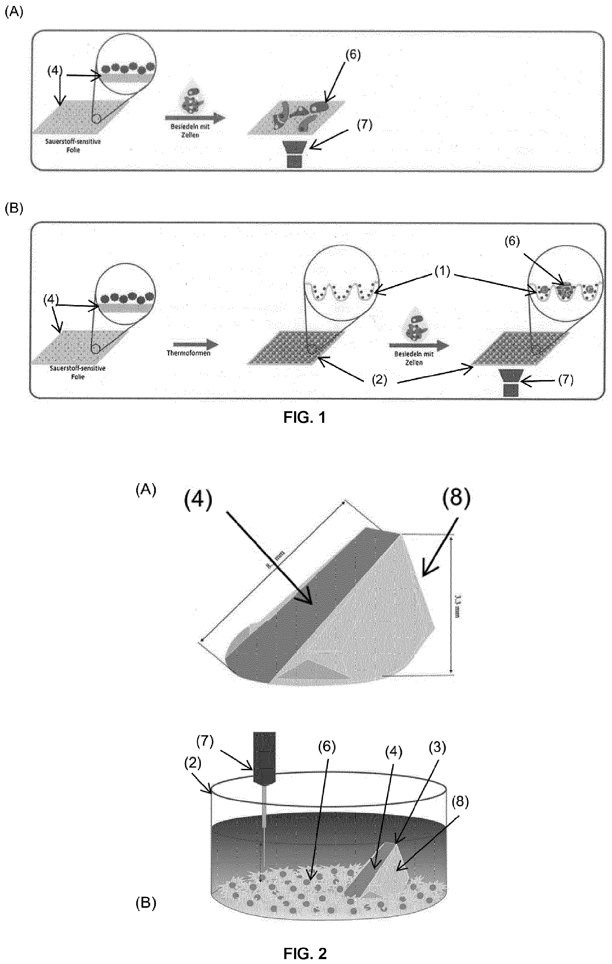

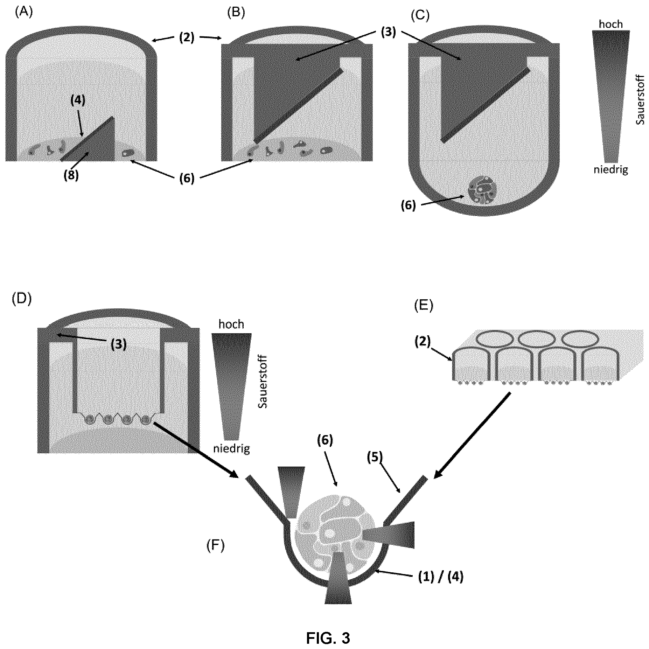

Figur 2 illustriert eine solche Rampe mit einer planaren Sensorfolie (Fig. 2A) und deren Anordnung in einem zweidimensionalen Zellkultursystem (Fig. 2B). Die zweidimensionalen Sensorfolien sind hierbei planar und auf Haltemittel in Form von Rampen aufgebracht. Diese makroskopischen Haltemittel (in der Größenordnung von einigen cm) werden bspw. gedruckt oder gefräst und die planaren Sauerstoff-sensitiven Folien anschließend aufgeklebt. Die eigentliche Zellkultur erfolgt in herkömmlichen Zellkulturgefäßen wie Petrischalen, vorwiegend jedoch in Multiwellplatten mit 6, 8, 12, 24, 48 oder 96 Wells. Figur 3A bis 3C illustrieren solche Systeme gemäß dem Stand der Technik. Zumeist werden solche Systeme als zweidimensionale Zellkultur in Form eines Zell-Monolayers auf dem Boden der Kultivierungsmaterialien realisiert. Die Rampe zur Sauerstoffmessung wird in dieses bestehende System eingebracht, indem sie entweder auch auf dem Boden befestigt wird (Fig. 3A) oder in Wells der Multiwellplatte eingehängt wird (Fig. 3B). Derartige Folien eignen sich jedoch aufgrund ihrer planaren Form nicht für Messungen in dreidimensionalen Zellkulturen. Grundsätzlich wäre auch denkbar, wenn auch bisher nicht beschrieben, derartige bedruckte Rampen in Multiwellplatten mit runden, U-förmigen Böden einzuhängen, um so die Kultivierung von Zellaggregaten zu ermöglichen (Fig. 3C). Nachteilig an einem solchen System wäre aber weiterhin, dass hier lediglich der Sauerstoffgradient entlang der z-Achse und in einem vergleichsweise großen Abstand zur Zelle oder zum Zellaggregat messbar ist.

## AUFGABENSTELLUNG

Die Aufgabe der vorliegenden Erfindung bestand darin, neue und verbesserte Systeme und Verfahren zur Messung von Analyten und Analytgradienten, insbesondere von Sauerstoff, in Zellkulturen bereitzustellen, die die Nachteile der aus dem Stand der Technik bekannten Systeme und Verfahren nicht aufweisen. Die neuen Systeme und Verfahren sollen insbesondere zur Analytmessung in der direkten Mikroumgebung dreidimensionaler Zellkulturen geeignet sein. Außerdem sollen die neuen und verbesserten Systeme und Verfahren besonders die Bestimmung von Analytgradienten ermöglichen. In einem weiteren Aspekt der Erfindung sollen die neuen und verbesserten Systeme und Verfahren die simultane Zellkultivierung und Analytmessung ermöglichen. Darüber hinaus soll es mit den verbesserten Systemen und dem neuentwickelten Messverfahren möglich sein, große Datenmengen in einem Hochdurchsatzverfahren aufzunehmen und Messwerte über einen langen Zeitraum zu generieren.

In einem weiteren Aspekt der Erfindung lag die Aufgabe darin, neue und verbesserte Systeme bereitzustellen, worin eine Art planare Rampe in einer dreidimensionalen Struktur integriert bzw. darin simuliert werden kann.

Die Erfinder der vorliegenden Erfindung fanden überraschend, dass diese Aufgaben gelöst werden konnten, indem zweidimensionale Sensorfolien zu dreidimensionalen Strukturen geformt werden, beispielsweise mittels Mikrothermoformverfahren, mit dem sich die Möglichkeit ergibt, solche Sensorfolien zu sogenannten Mikrokavitätenarrays aus Analyt-sensitiven Folien zu formen. Es wurde überraschend gefunden, dass solche dreidimensionalen Strukturen aus funktionalisierten Sensorfolien selbst zur Aufnahme und Kultivierung von Zellkulturen geeignet sind und dadurch die Analytmessung in deutlich geringerer Entfernung zum Zellgewebe möglich wird. Im Vergleich zu den bisher bekannten und kommerziell erhältlichen Folien wird dadurch nicht nur die Messung von Analyten wie Sauerstoff in 3D-Zellkulturen ermöglicht, sondern die Folien stellen auch noch selbst das Kultursystem dar.

Durch die zusätzliche Ausbildung einer Fase an der Mikrostruktur kann darüber hinaus auch eine Art Rampe in die Struktur integriert werden. Durch eine solche erfindungsgemäße Art der Integration einer Fase können Tiefenprofilinformationen erhalten und somit Analytgradienten gemessen werden. Betrachtet man derartige erfindungsgemäße Fasen unabhängig von den erfindungsgemäßen Kavitäten, so entsprechen diese vereinfacht dargestellt den schiefen Ebenen ("Schrägen") der Rampen aus dem Stand der Technik, werden aber - anders als im Stand der Technik - genauso wie die erfindungsgemäßen Kavitäten aus der funktionalisierten Sensorfolie gebildet, anstatt mit einer solchen beklebt zu werden. Obwohl also sowohl bei den aus dem Stand der Technik bekannten Verfahren als auch bei den erfindungsgemäßen dreidimensionalen Strukturen Schrägen oder schiefe Ebenen für die Messung von Analyt-Gradienten entlang der z-Achse verwendet werden, unterscheidet sich der erfindungsgemäße Ansatz bereits aus den vorstehend genannten Gründen signifikant von dem gemäß des Stands der Technik.

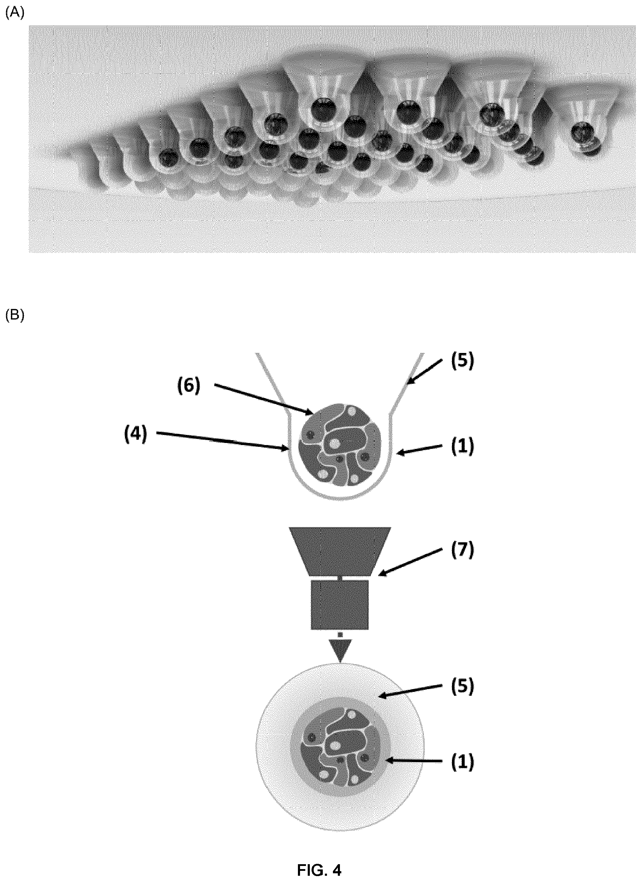

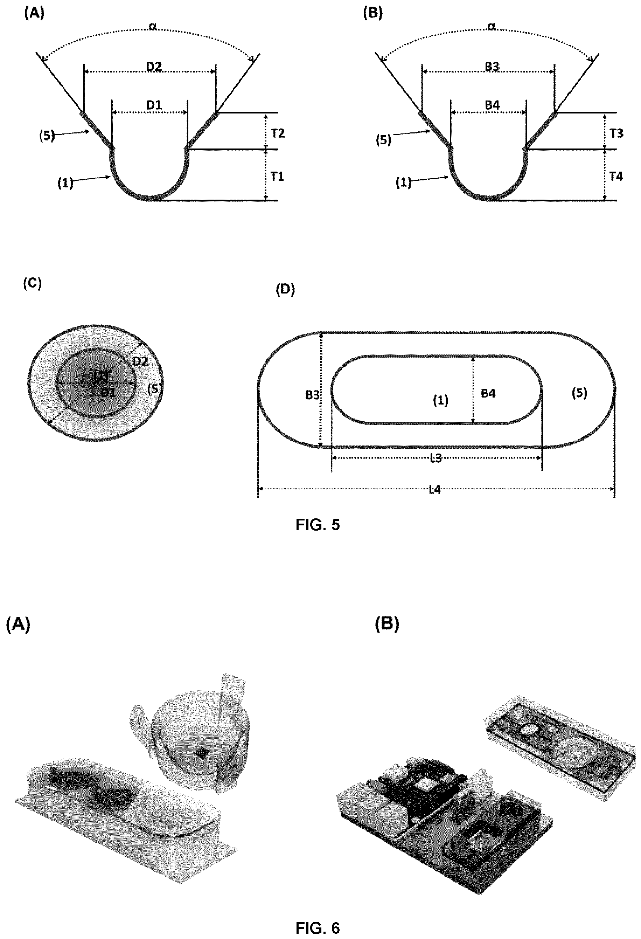

Darüber hinaus dient in den erfindungsgemäßen Strukturen mit Fase (siehe z.B. Fig. 4A) - im Gegensatz zu den Rampen-Inserts aus dem Stand der Technik - die mikrostrukturierte funktionalisierte Analyt-sensitive Folie selbst als Zellkultivierungssystem. Durch das Mikrothermoformen der Folie in bspw. Mikrokavitäten können direkt in der Sensorfolie die Zellen dreidimensional kultiviert werden. Dies ermöglicht auch, dass 3D-Zellkulturen direkt in Form sogenannter Sphäroide in den Mikrokavitäten generiert werden können. Durch eine Plasmabehandlung oder durch Beschichtung der erfindungsgemäßen Mikrokavitätenarrays mit geeigneten Beschichtungsmitteln, beispielsweise einem anti-adhäsiven Beschichtungsmittel, z.B. einem kommerziell erhältlichen Film BIOFLOAT™ der Firma FaCellitate GmbH, kann die Kultivierung von Zellsphäroiden bzw. dreidimensionalen Zellaggretaten positiv beeinflusst werden. Die Mikrokavitäten haben üblicherweise einen geringen Durchmesser, sodass auf der Fläche bspw. eines Wells einer 96-Well-Platte mehrere hundert bis tausend Mikrokavitäten generiert und somit auch mehrere Hundert bis Tausend Sphäroide kultiviert werden können. Für jeden einzelnen dieser Sphäroide können Informationen über die Analytgradienten gewonnen werden. Messungen in dieser Größenordnung sind mit den im Stand der Technik beschriebenen Ansätzen in der Praxis nicht umsetzbar. Die erfindungsgemäßen Analyt-sensitiven Mikrokavitäten können für eine bessere Handhabung bspw. an Zellkultur-Inserts gebondet werden (Fig. 3D), die in Multiwellplatten eingehängt werden können. Für Hochdurchsatzexperimente ist es auch möglich den Boden von Multiwellplatten durch Folien mit Mikrokavitäten zu ersetzen (Fig. 3E). Wie bereits beschrieben, können in den einzelnen Mikrokavitäten dann Analytgradienten entlang der z-Achse, also unmittelbar oberhalb des Sphäroids (Fig. 3F), gemessen werden. Weitere Informationen über Analytkonzentrationen in der Mikroumgebung der Zellen lassen sich ebenso gewinnen, da über die gesamte Fläche der Mikrokavität gemessen werden kann. Für die Bestimmung von Analytgradienten wird dabei eine Fase im oberen Bereich der Mikrostrukturen geformt (Fig. 4A, Fig. 5). Dadurch können in der zweidimensionalen Aufnahme durch Messung an verschiedenen Punkten Informationen entlang der Fase und somit zu unterschiedlichen Tiefen gewonnen werden (Fig. 4B). Ferner ermöglicht der hier geschilderte Aufbau, Zellkulturen über einen längeren Zeitraum direkt im Messaufbau zu lagern und automatisierte Langzeitstudien auszuführen, da unter anderem die genaue Position der Sphäroide durch die Mikrokavitätenarrays/Sensorarrays gegeben ist und automatisiert so gezielt einzelne Sphäroide beobachtet werden können.

Im Vergleich zu den im Stand der Technik beschriebenen Ansätzen können so Informationen aus allen drei Dimensionen gewonnen werden, die durch die derzeit verwendeten Auswertesysteme, in einer zweidimensionalen Darstellung zusammengefasst werden. Durch die Verwendung von Konfokalmikroskopen ist jedoch auch eine dreidimensionale Darstellung und Auswertung realisierbar.

## BESCHREIBUNG DER ERFINDUNG

Die vorliegende Erfindung wird nachfolgend näher beschrieben und umfasst insbesondere die folgenden Aspekte:

[1] Dreidimensionale Strukturen, die aus funktionalisierten Sensorfolien gebildet sind.

[2] Funktionalisierte Sensorfolien in Form von dreidimensionalen Strukturen zur Aufnahme und/oder Züchtung von Zellen.

[3] Dreidimensionale Strukturen nach [1] oder funktionalisierte Sensorfolien nach [2], wobei es sich bei den Strukturen um Mikrostrukturen oder Mikrokavitäten handelt.

[4] Funktionalisierte Sensorfolien nach [2], welche mikrostrukturierte funktionalisierte Sensorfolien sind, zur simultanen Zellkultur und Analytmessung, insbesondere zur Messung von $O_2$, $CO_2$, Glukose und/oder pH-Wert.

[5] Dreidimensionale Strukturen oder funktionalisierte Sensorfolien nach [1] bis [4], welche in Form von Mikrokavitätenarrays, Mikrokanälen oder anderen Mikrovertiefungen für die Anwendung (den Einsatz) in Mikrotiterplatten, in Zellkulturplatten oder in Zellkultur-Inserts mit einem oder mehreren Kompartimenten vorliegen.

[6] Dreidimensionale Strukturen oder funktionalisierte Sensorfolien nach [1] bis [5], worin die Vertiefungen der einzelnen (Mikro)Kavitäten einen Durchmesser bis 4.000 $\mu$m, bevorzugt bis 2.000 $\mu$m, bevorzugter bis 1.000 $\mu$m, bevorzugter bis 800 $\mu$m, bevorzugter bis 500 $\mu$m und/oder einen Durchmesser von mindestens 10 $\mu$m, bevorzugter mindestens 50 $\mu$m, bevorzugter mindestens 100 $\mu$m, bevorzugter mindestens 200 $\mu$m, noch bevorzugter mindestens 300 $\mu$m aufweisen, wobei Durchmesser im Bereich von 1 bis 4.000 $\mu$m, oder 10 bis 2.000 $\mu$m, oder 100 bis 1.000 $\mu$m, oder 200 bis 800 $\mu$m, oder 300 bis 500 $\mu$m möglich sind.

[7] Dreidimensionale Strukturen oder funktionalisierte Sensorfolien nach [1] bis [5], worin kanalförmige Strukturen eine Kanallänge von 120 mm, bevorzugter 60 mm, noch bevorzugter 20 mm aufweisen.

[8] Dreidimensionale Strukturen oder funktionalisierte Sensorfolien nach [7], worin kanalförmige Strukturen eine Kanalbreite bis 4.000 $\mu$m, bevorzugt bis 2.000 $\mu$m, bevorzugter bis 1.000 $\mu$m, bevorzugter bis 800 $\mu$m, bevorzugter bis 500 $\mu$m und/oder eine Breite von mindestens 10 $\mu$m, bevorzugter mindestens 50 $\mu$m, bevorzugter mindestens 100 $\mu$m, be-

vorzugter mindestens 200 μm, noch bevorzugter mindestens 300 μm aufweisen, wobei Breiten im Bereich von 1 bis 4.000 μm, oder 10 bis 2.000 μm, oder 100 bis 1.000 μm, oder 200 bis 800 μm, oder 300 bis 500 μm möglich sind.

[9] Dreidimensionale Strukturen oder funktionalisierte Sensorfolien nach [1] bis [8], worin mehrere Kavitäten oder kanalförmige Strukturen unabhängig voneinander gleiche oder unterschiedliche Dimensionen (Längen, Breiten, Durchmesser, Tiefen) aufweisen und auf einer Fläche von $120 \times 80$ mm$^2$ unter Bildung einer Multikavitäten-Struktur angeordnet sind.

[10] Dreidimensionale Strukturen oder funktionalisierte Sensorfolien nach [1] bis [9], welche mindestens 1 bis 1.000.000 Mikrokavitäten/cm$^2$ aufweisen, bevorzugt zwischen 10 und 10.000 Mikrokavitäten/cm$^2$, noch bevorzugter zwischen 100 und 1000 Mikrokavitäten/cm$^2$, welche in gerader Linie oder zueinander versetzt angeordnet sein können.

[11] Dreidimensionale Strukturen oder funktionalisierte Sensorfolien nach [1] bis [10], worin kanalförmige Strukturen geradlinig, verzweigt oder mäanderförmig angeordnet sein können.

[12] Dreidimensionale Strukturen oder funktionalisierte Sensorfolien nach [1] bis [11], worin die Tiefe der einzelnen Kavität durch ein Aspektverhältnis (DurchmesserTiefe) von 1:2, bevorzugt 1:1 noch bevorzugter 2:1 charakterisiert ist.

[13] Dreidimensionale Strukturen oder funktionalisierte Sensorfolien nach [1] bis [12], worin die funktionalisierten Sensorfolien fluoreszierende Folien sind.

[14] Dreidimensionale Strukturen oder funktionalisierte Sensorfolien nach [1] bis [13], worin die funktionalisierten Sensorfolien Fluorophor-dotierte (beschichtete) Folien sind.

[15] Dreidimensionale Mikrostrukturen oder funktionalisierte Sensorfolien nach [1] bis [14], worin die funktionalisierten Sensorfolien dotierte Polymerträgerfolien oder Polycarbonatträgerfolien sind, wobei die Trägerfolien bevorzugt eine Dicke von bis zu 100 μm, bevorzugter bis zu 50 μm aufweisen.

[16] Dreidimensionale Strukturen oder funktionalisierte Sensorfolien nach [1] bis [15], worin die Fluorophor-Beschichtung (Dotierung) in Form von auf die Trägerfolien aufgebrachten oder in Form von in Hydrogelen oder in Polymermatrizes immobilisierten Fluorophoren vorliegt.

[17] Dreidimensionale Strukturen oder funktionalisierte Sensorfolien nach [1] bis [16], worin die funktionalisierten Sensorfolien Fluorophor-dotierte (beschichtete) Folien sind und die Fluorophore ausgewählt sind aus der Gruppe der Metallkomplexe, umfassend insbesondere Platin-II-, Palladium-II- und Ruthenium-II-Komplexe.

[18] Dreidimensionale Strukturen oder funktionalisierte Sensorfolien nach [1] bis [17], welche Analytsensitive Sensorfolien sind.

[19] Dreidimensionale Strukturen oder funktionalisierte Sensorfolien nach [1] bis [18], worin die funktionalisierten Sensorfolien aus Sauerstoff-, $CO_2$-, Glukose- oder pH-sensitiven Sensorfolien ausgewählt sind.

[20] Dreidimensionale Strukturen oder funktionalisierte Sensorfolien nach [1] bis [19], worin die funktionalisierten Sensorfolien Sauerstoff-sensitive Sensorfolien sind.

[21] Dreidimensionale Strukturen oder funktionalisierte Sensorfolien nach [1] bis [20], worin die Kavitäten eine Fase an der Öffnung der Kavitäten aufweisen.

[22] Dreidimensionale Strukturen oder funktionalisierte Sensorfolien nach [21], worin die Fasen einen Öffnungswinkel von 1 bis 179°, bevorzugt von 40 bis 120°, noch bevorzugter von 60 bis 90° aufweisen.

[23] Dreidimensionale Strukturen oder funktionalisierte Sensorfolien nach [1] bis [22], die zusätzlich einen oder mehrere Bereiche aufweisen, welche eine zusätzliche Funktionalisierung aufweisen.

[24] Dreidimensionale Strukturen oder funktionalisierte Sensorfolien nach [23], worin die zusätzlichen funktionalisierten Bereiche in Form von Spots ausgebildet sind, die eine Sauerstoff-, $CO_2$-, Glukose- oder pH-Sensitivität aufweisen, und die räumlich getrennt von den funktionalisierten Mikrokavitäten angeordnet sind.

[25] Dreidimensionale Strukturen oder funktionalisierte Sensorfolien nach [1] bis [24], worin die die Mikrokavitäten bildenden funktionalisierten Sensorfolien mittels einer Plasmabehandlung und/oder durch Beschichtung mit Beschichtungsmitteln, wie Collagen, modifiziert sind.

[26] Fluidische Systeme oder Mikrobioreaktoren umfassend die dreidimensionalen Strukturen oder funktionalisierten Sensorfolien nach [1] bis [25] sowie optional einen oder mehrere Bereiche, welche eine zusätzliche Funktionalisierung aufweisen, wobei die zusätzlichen funktionalisierten Bereiche bevorzugt in Form von Spots ausgebildet sind, die eine Sauerstoff-, CO₂-, Glukose- oder pH-Sensitivität aufweisen, und die räumlich getrennt von den funktionalisierten Mikrokavitäten angeordnet sein können.

[27] Verfahren zur Herstellung Analyt-sensitiver dreidimensionaler Strukturen wie in [1] bis [25] definiert, umfassend die Schritte

- Bereitstellung funktionalisierter Sensorfolien, insbesondere solcher wie vorstehend beschrieben;
- Verformung der funktionalisierten Sensorfolien mittels M ikrothermoformverfahren;
- und Ausbildung der Analyt-sensitiven dreidimensionalen Strukturen.

[28] Verfahren nach [27], worin die Analyt-sensitiven dreidimensionalen Strukturen in Form von Mikrokavitätenarrays, Mikrokanälen oder anderen Mikrovertiefungen für die Anwendung (den Einsatz) in Mikrotiterplatten, in Zellkulturplatten oder in Zellkultur-Inserts mit einem oder mehreren Kompartimenten ausgebildet werden.

[29] Verfahren nach [27] bis [28], umfassend des Weiteren die Schritte

- Herstellung oder Bereitstellung einer Formmaske mit einer Negativform der dreidimensionalen kavitätischen Mikrostruktur, umfassend eine oder mehrere Kavitäten sowie optional eine Fase an der Öffnung der Kavitäten;
- Abformen der funktionalisierten Sensorfolien in der Maske;
- Erhalt der Analyt-sensitiven dreidimensionalen Strukturen, insbesondere solcher in Form von (Mikro)Kavitäten, welche gegebenenfalls eine Fase an der Kavitätenöffnung aufweisen.

[30] Verfahren nach [27] bis [29], umfassend außerdem die

- Aufbringung zusätzlicher funktionalisierter Bereiche, und/oder
- Behandlung der die Mikrokavitäten bildenden funktionalisierten Sensorfolien mit Plasma und/oder Beschichtung mit Beschichtungsmitteln, wie anti-adhäsiven Beschichtungsmitteln oder extrazellulärer Matrix (z.B. Collagen).

[31] Verfahren zur (non-invasiven) Messung von Analyten oder Analytgradienten in einer Zellkultur, umfassend

a) Zellkultivierung in den Analyt-sensitiven dreidimensionalen Strukturen wie in [1] bis [25] oder in den Fluidischen Systemen oder Mikrobioreaktoren gemäß [26] definiert;
b) Analytmessung mittels Mikroskopie, beispielsweise Konfokalmikroskopie, insbesondere Fluoreszenzlebenszeit-Mikroskopie;
c) automatisierte Erfassung und Auswertung der Messdaten.

[32] Verfahren gemäß [31], worin die Analytmessung an verschiedenen Messpunkten (ortsaufgelöst) unter Erfassung des Analytgradienten erfolgt.

[33] Verfahren gemäß [31] bis [32], zur Bestimmung von Sauerstoff-, CO₂-, Glukose-Konzentrationen oder pH-Werten oder deren Gradienten.

[34] Verfahren gemäß [31] bis [33], worin die Zellkultur eine dreidimensionale Zellkultur ist und in Schritt a) eine dreidimensionale Zellkultivierung erfolgt.

[35] Verfahren gemäß [31] bis [34], welches dadurch gekennzeichnet ist, dass es Hydrogel-frei und ohne Verwendung von Nanopartikel-gebundenen Fluorophoren durchgeführt wird.

[36] Verwendung der dreidimensionalen Strukturen oder funktionalisierten Sensorfolien nach [1] bis [25] oder der Fluidischen Systeme oder Mikrobioreaktoren gemäß [26] zur simultanen Zellkultur und Analytmessung, insbesondere zur Messung von O₂, CO₂, Glukose, pH-Werten und Gradienten davon.

[37] Verwendung der dreidimensionalen Strukturen oder funktionalisierten Sensorfolien nach [1] bis [25] oder der Fluidischen Systeme oder Mikrobioreaktoren gemäß [26] zur Kultivierung von Zellen, worin die Zellen primäre Zellen, Zelllinien, Stammzellen und jeweils daraus hervorgegangener gentechnisch veränderter Zellen, wie z.B. auch Chimäre Antigenrezeptor-T-Zellen (CAR-T-Zellen) umfassen.

[38] Verwendung der dreidimensionalen Strukturen oder funktionalisierten Sensorfolien nach [1] bis [25] oder der Fluidischen Systeme oder Mikrobioreaktoren gemäß [26] in Verfahren zur Charakterisierung von Zellkulturbedingungen, zur Entwicklung Zell-basierter Modelle für Metabolismusstudien, zur Testung von Wirkstoffen, zur Testung der (ischämischen) Toxikologie *in vitro*.

[39] Verwendung der dreidimensionalen Strukturen oder funktionalisierten Sensorfolien nach [1] bis [25] oder der Fluidischen Systeme oder Mikrobioreaktoren gemäß [26] für die in [36] bis [38] definierten Verwendungen im Hochdurchsatzverfahren.

[40] Verwendung der dreidimensionalen Strukturen oder funktionalisierten Sensorfolien nach [1] bis [25] oder der Fluidischen Systeme oder Mikrobioreaktoren gemäß [26] für die in [36] bis [38] definierten Verwendungen in Langzeitstudien.

[41] Vorrichtung umfassend

- die dreidimensionalen Strukturen oder funktionalisierten Sensorfolien nach [1] bis [25] oder die Fluidischen Systeme oder Mikrobioreaktoren gemäß [26] zur Zellkultivierung und Probenaufnahme,
- ein Detektionselement zur Erfassung der Messdaten von der funktionalisierten Sensorfolie, insbesondere ein Mikroskop mit Sensor oder Detektionseinrichtung, beispielsweise eine Kamera, insbesondere ein Konfokalmikroskop,
- ein Element zur automatisierten Erfassung von Messdaten, Datenverarbeitung, Datenauswertung und Datenausgabe.

[42] Vorrichtung gemäß [41], worin die dreidimensionalen Strukturen oder funktionalisierten Sensorfolien durch die Merkmale gemäß [1] bis [25] charakterisiert sind.

## DETAILLIERTE BESCHREIBUNG DER ERFINDUNG

Im Sinne der vorliegenden Erfindung bezeichnet der Begriff "dreidimensionale Strukturen" zur Charakterisierung der funktionalisierten Sensorfolien insbesondere "dreidimensionale Mikrostruktur(en)" und dabei solche Formen bzw. Formkörper, die eine Vertiefung, Höhlung, einen Hohlraum oder eine sonstige Form zur Aufnahme eines Volumens ausbilden. Die erfindungsgemäßen Strukturen können im Grunde jede geometrische Form aufweisen, bevorzugt sind diese rund bzw. halbrund, kugelig oder weisen die Form von langgezogenen Kanälen, jeweils vorzugsweise mit nach außen gewölbtem Boden, auf.

Die erfindungsgemäßen Strukturen sind zur Aufnahme und/oder Kultur von Zellen vorgesehen. Die erfindungsgemäßen dreidimensionalen Strukturen weisen daher eine Öffnung auf, die die Aufnahme von Zellen, Kulturmedien, sowie sonstige Reagenzien zur Zellkultur und -analyse etc. ermöglicht. Zelltypen, die in den erfindungsgemäßen dreidimensionalen Strukturen kultiviert werden können, umfassen beispielsweise HepG2- oder HeLa-Zellen oder Murine P19- oder aus menschlichen Stammzellen abgeleitete Kardiomyozyten. Mögliche Mikrostrukturen sind beispielhaft in den Figuren 4A, 5A und 5B illustriert.

Die dreidimensionalen Strukturen der vorliegenden Erfindung werden aus funktionalisierten Sensorfolien gebildet bzw. geformt. Die Erfindung betrifft somit auch funktionalisierte Sensorfolien in Form von dreidimensionalen Strukturen, insbesondere Mikrostrukturen zur Aufnahme und/oder Kultur von Zellen. Im Gegensatz zum vorstehend beschriebenen Stand der Technik liegen die funktionalisierten Sensorfolien somit nicht in Form einer planaren (d.h. zweidimensionalen) Schicht oder Folie vor, sondern werden in eine dreidimensionale Form überführt, die einen Hohlraum oder eine Wölbung zur Aufnahme der Zellen ausbildet. Die erfindungsgemäßen Sensorfolien unterscheiden sich damit von den im Stand der Technik eingesetzten Sensorfolien.

Je nach Größe der Strukturen können diese in bevorzugten Ausführungsformen auch als sogenannte Mikrostrukturen oder Mikrokavitäten vorliegen.

Die erfindungsgemäßen dreidimensionalen Strukturen oder funktionalisierten Sensorfolien werden bevorzugt in Form von Mikrokavitätenarrays, Mikrokanälen oder anderen Mikrovertiefungen ausgebildet, und liegen damit insbesondere in bzw. als Mikrotiterplatten, Zellkulturplatten oder in Zellkultur-Inserts mit einem oder mehreren Kompartimenten vor.

Im Fall von erfindungsgemäß bevorzugten Mikrostrukturen handelt es sich insbesondere um Mikrokavitäten, welche in Form runder bzw. halbrunder oder kanalförmiger Vertiefungen in der Sensorfolie ausgebildet sind. Bevorzugter verfügen diese außerdem im oberen Bereich über eine Fase, wie nachfolgend detaillierter beschrieben. In bevorzugten möglichen Ausführungsformen sind diese Mikrokavitäten als Arrays (Mikrokavitätenarray) angeordnet.

Bezüglich der Dimensionen der erfindungsgemäßen Kavitäten bezeichnet "Tiefe" den längsten Abstand vom Boden einer Kavität bis zu deren Rand ohne Fase (T2 in Fig. 5A bzw. T4 in Fig. 5B). Runde/halbrunde Kavitäten können durch ihren Durchmesser an der oberen Öffnung ohne Fase (D1 in Fig. 5A) charakterisiert werden, wohingegen bei kanalförmigen Kavitäten die "Breite" den kürzeren Abstand zwischen zwei Seitenwänden ohne Fase (B4 in Fig. 5B) im Gegensatz zu deren 'Länge' mit dem längsten Abstand zwischen zwei Seitenwänden ohne Fase (L3 in Fig. 5B) bezeichnet.

Je nach Dimensionierung der Kavitäten handelt es sich dann um Kavitäten oder Mikrokavitäten.

Die einzelnen (Mikro)Kavitäten der erfindungsgemäßen dreidimensionalen Strukturen oder funktionalisierten Sensorfolien können einen Durchmesser (gemessen am oberen Rand der Kavität ohne Fase) bis 4.000 $\mu$m, bevorzugt bis 2.000 $\mu$m, bevorzugter bis 1.000 $\mu$m, bevorzugter bis 800 $\mu$m, bevorzugter bis 500 $\mu$m aufweisen.

Die einzelnen (Mikro)Kavitäten der erfindungsgemäßen dreidimensionalen Strukturen oder funktionalisierten Sensorfolien können einen Durchmesser (gemessen am oberen Rand der Kavität ohne Fase) von mindestens 10 $\mu$m, bevorzugter mindestens 50 $\mu$m, bevorzugter mindestens 100 $\mu$m, bevorzugter mindestens 200 $\mu$m, noch bevorzugter mindestens 300 $\mu$m aufweisen.

Durchmesser im Bereich von 1 bis 4.000 $\mu$m, oder 10 bis 2.000 $\mu$m, oder 100 bis 1.000 $\mu$m, oder 200 bis 800 $\mu$m, oder 300 bis 500 $\mu$m sind möglich.

Liegen die erfindungsgemäßen dreidimensionalen Strukturen oder funktionalisierten Sensorfolien in Form kanalförmiger Strukturen vor, so können diese eine Kanallänge (gemessen in der Kavität ohne Fase) von 120 mm, bevorzugter 60 mm, noch bevorzugter 20 mm aufweisen.

Erfindungsgemäße dreidimensionale Strukturen oder funktionalisierte Sensorfolien können eine Kanalbreite (gemessen in der Kavität ohne Fase) bis 4.000 $\mu$m, bevorzugt bis 2.000 $\mu$m, bevorzugter bis 1.000 $\mu$m, bevorzugter bis 800 $\mu$m, bevorzugter bis 500 $\mu$m aufweisen.

Erfindungsgemäße dreidimensionale Strukturen oder funktionalisierte Sensorfolien können eine Kanalbreite (gemessen in der Kavität ohne Fase) von mindestens 10 $\mu$m, bevorzugter mindestens 50 $\mu$m, bevorzugter mindestens 100 $\mu$m, bevorzugter mindestens 200 $\mu$m, noch bevorzugter mindestens 300 $\mu$m aufweisen.

Kanalbreiten im Bereich von 1 bis 4.000 $\mu$m, oder 10 bis 2.000 $\mu$m, oder 100 bis 1.000 $\mu$m, oder 200 bis 800 $\mu$m, oder 300 bis 500 $\mu$m sind möglich.

Bevorzugt werden mehrere der erfindungsgemäßen dreidimensionalen Strukturen oder funktionalisierten Sensorfolien in Nachbarschaft zueinander auf einer Fläche angeordnet, so dass mehrere Kavitäten oder kanalförmige Strukturen mit gleichen oder unterschiedlichen Formen und/oder Dimensionen (d.h. gleichen oder unterschiedlichen Längen, Breiten, Durchmessern, Tiefen und runden bzw. kanalförmigen Formen) nebeneinander vorliegen und auf einer Fläche, z.B. einer Fläche von 120 $\times$ 80 mm$^2$, eine sogenannte Multikavitäten-Struktur bilden. Liegen in derartigen Multikavitäten-Strukturen die einzelnen Kavitäten in Form kanalförmiger Kavitäten vor, so können diese grundsätzlich geradlinig, verzweigt oder mäanderförmig angeordnet sein.

Die Tiefe der erfindungsgemäßen dreidimensionalen Strukturen oder funktionalisierten Sensorfolien kann durch das Aspektverhältnis (Durchmesser : Tiefe) der einzelnen Kavität charakterisiert werden, wobei das Aspektverhältnis der einzelnen Kavitäten bevorzugt 1:2, bevorzugter 1:1, noch bevorzugter 2:1 beträgt.

Die einzelnen (Mikro)Kavitäten der erfindungsgemäßen dreidimensionalen Strukturen oder funktionalisierten Sensorfolien können eine Gesamttiefe bis 800 $\mu$m, bevorzugt bis 700 $\mu$m, bevorzugter bis 600 $\mu$m, noch bevorzugter bis 500 $\mu$m, noch bevorzugter bis 450 $\mu$m und/oder eine Gesamttiefe von mindestens 100 $\mu$m, bevorzugt mindestens 200 $\mu$m, bevorzugter mindestens 250 $\mu$m aufweisen, wobei eine Gesamttiefe im Bereich von 100 bis 600 $\mu$m, 200 bis 500 $\mu$m, oder 250 bis 450 $\mu$m ganz besonders bevorzugt sind.

Besonders bevorzugte runde Kavitäten lassen sich wie folgt charakterisieren:
Die Form der einzelnen Kavität (1) entspricht einer gerundeten, geöffneten Form, beispielsweise einer Halbkugel oder (bei größeren Durchmessern) einer Form, die flacher als eine Halbkugel ist oder (bei kleineren Durchmessern) einer Form, die nach der Halbkugel noch einen senkrechten Bereich aufweiset. Die Tiefe der einzelnen Kavität (T1) ist im Optimalfall $T1 = \frac{D1}{2}$, wobei generell $0 < T1 \leq D1$ möglich ist. Noch tiefere Kavitäten sind denkbar, aber für Zellkulturanwendungen eher unrelevant. Für den Durchmesser (D2) der Fase (5) gilt $D2 > D1$, für die Tiefe (T2) gilt $T2 > 0$. Aus D2 und T2 ergibt sich dann auch der Öffnungswinkel $\alpha$. Größere Durchmesser für D1 als 4000 $\mu$m sind technisch möglich, für den Bereich der Zellkultur aber nur bedingt relevant, zudem würde man hier dann eher von Makrokavitäten sprechen. Durchmesser von bis zu 1 $\mu$m sind mit dünneren Folien denkbar, aber nur für Anwendungen außerhalb der Zellkultur sinnvoll.

Besonders bevorzugte kanalförmige Kavitäten, optional auch mit Fase, lassen sich wie folgt charakterisieren:
Die Größenordnungen sind vergleichbar mit denen der vorstehend beschriebenen bevorzugten runden Kavitäten, wobei statt des Durchmessers die Breite des Kanals (B4) definiert wird, welche 10 bis 4000 $\mu$m betragen kann. Da der Kanalquerschnitt im Optimalfall halbrund ist, gilt für die Tiefe $T4 = \frac{D4}{2}$, wobei generell $0 < T4 \leq D4$ möglich ist. Die Fase kann wie bei der runden Kavität gestaltet sein. Kanallängen (L3) liegen üblicherweise im Bereich bis zu 40 mm, längere und auch verzweigte Kanäle sind denkbar und möglich.

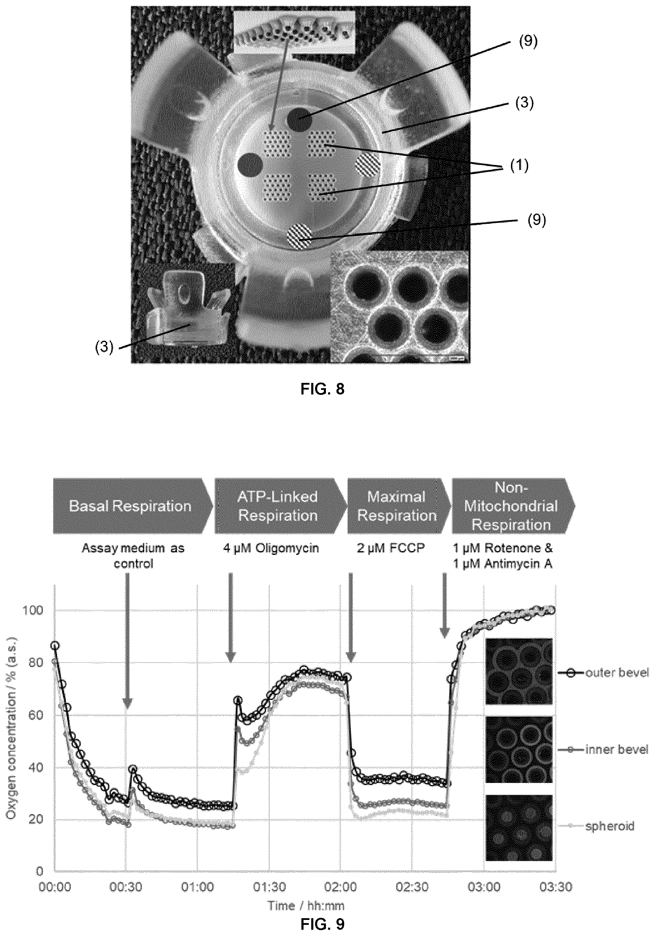

Die Mikrokavitäten können einzeln vorliegen oder in Mikrokavitätenarrays angeordnet sein, wobei sich darin die einzelnen Kavitäten berühren oder auch über einen Abstand verfügen können. Ein einzelnes Array verfügt über zumindest eine Mikrokavität, die in Spalten und Zeilen mit verschiedener Anzahl angeordnet sein können. Üblicherweise werden Arrays mit 30 bis 1000 Mikrokavitäten verwendet. Solche Anordnungen können sich auch auf Folien im Format einer Multiwellplatte erstrecken, sodass in einem Abformvorgang je Folie bis zu 20.000 Mikrokavitäten generiert werden können. Derartige Multikavitäten-Strukturen können bis zu 1.000.000 Mikrokavitäten pro cm$^2$ aufweisen, bevorzugt weisen solche Multikavitätenstrukturen mindestens 1 bis 1.000.000 Mikrokavitäten/cm$^2$ auf, bevorzugt zwischen 10 und 10.000 Mikrokavitäten/cm$^2$, noch bevorzugter zwischen 100 und 1000 Mikrokavitäten/cm$^2$. Diese können dabei in gerade Linie, parallel nebeneinander oder zueinander versetzt angeordnet sein. Die Multikavitätenstrukturen können über die gesamte Fläche gleichmäßig verteilt vorliegen oder nur in bestimmten Bereichen vorgesehen werden und z.B. als "Gruppen" organisiert angeordnet werden. Eine solche beispielhafte gruppierte Anordnung illustriert Figur 8.

Da die erfindungsgemäßen dreidimensionalen Strukturen aus funktionalisierten Sensorfolien geformt werden, handelt es sich im Grunde genommen bei diesen um (mikro)strukturierte funktionalisierte Sensorfolien, die aufgrund der erfindungsgemäßen Verformung zu dreidimensionalen Strukturen zur simultanen Zellkultur und Analytmessung, wie insbesondere von $O_2$, $CO_2$, Glukose und/oder pH-Wert geeignet sind. Dabei dient die "Messvorrichtung", nämlich die strukturierte Sensorfolie, selbst auch als Kultivierungsmaterial, was die Systeme der vorliegenden Erfindung grundlegend von den bekannten konventionellen Systemen unterscheidet.

Die funktionalisierten Sensorfolien, die zur Bildung / Formung der erfindungsgemäßen dreidimensionalen Strukturen verwendet werden, sind solche Folien worin ein geeigneter Träger derart dotiert, beschichtet oder in sonstiger Weise behandelt wird, dass eine Funktionalisierung erfolgt mittels derer gezielt analytische Messungen oder Bestimmungen möglich werden. Funktionalisierte Sensorfolien und deren Aufbau sind grundsätzlich bekannt.

Üblicherweise umfassen funktionalisierte Sensorfolien eine Trägerfolie, z.B. in Form von Polymerträgerfolien. Üblicherweise und bevorzugt weisen die Trägerfolien eine Dicke zwischen 1 $\mu$m und 100 $\mu$m, bevorzugter bis zu 50 $\mu$m auf.

Zur Bildung der erfindungsgemäßen Kavitäten sind insbesondere solche Sensorfolien von Interesse, die eine Funktionalisierung aufweisen mittels derer sich für die Zellkultur relevante Messparameter bestimmen und verfolgen lassen. Es handelt sich somit bei den funktionalisierten Sensorfolien der erfindungsgemäßen dreidimensionalen Strukturen insbesondere um Analyt-sensitive Sensorfolien.

Besonders relevante Messparameter umfassen die Messung und Bestimmung von $O_2$, $CO_2$, Glukose und/oder pH-Wert, wobei nicht nur die Erfassung von Einzelmesswerten relevant sind, sondern insbesondere auch Gradienten-Bestimmungen.

Die funktionalisierten Sensorfolien sind daher bevorzugt aus Sauerstoff-, $CO_2$-, Glukose- oder pH-sensitiven Sensorfolien ausgewählt, wobei Sauerstoff-sensitive Sensorfolien ganz besonders bevorzugt sind.

Entsprechend geeignete funktionalisierte Sensorfolien umfassen beispielsweise fluoreszierende Folien, insbesondere Fluorophor-dotierte Folien. Die Dotierung kann grundsätzlich in allen üblichen Formen erfolgen und beispielsweise in Form einer Beschichtung vorliegen. Es ist ebenfalls möglich, die Fluorophore in Hydrogelen oder in Polymermatrizes zu immobilisieren und in dieser Form auf die Trägerfolie aufzubringen.

Fluorophor-dotierte Sensorfolien umfassen bevorzugt solche, worin die Fluorophore ausgewählt sind aus der Gruppe umfassend Metallkomplexe, insbesondere solche aus der Gruppe der Platin-II-Porphyrine, Palladium-II-Porphyrine, Ruthenium-II-Komplexe, Platin-/Palladium-Komplexe sowie Iridium- und Europium-Komplexe.

In Zellkulturen ist die Bestimmung von Analytgradienten von besonderem Interesse.

Die Erfinder der vorliegenden Erfindung fanden überraschend, dass zur Bestimmung von Analytgradienten die Ausbildung einer sogenannten Fase an der Öffnung im oberen Bereich der kavitätischen Strukturen vorteilhaft ist. Wie bereits beschrieben, wird durch die zusätzliche Ausbildung einer solchen Fase am oberen Rand der Kavität eine Art Rampe oder schiefe Ebene in die Struktur integriert, an welcher in der zweidimensionalen Aufnahme durch Messung an verschiedenen Punkten Informationen entlang der Fase und somit zu unterschiedlichen Tiefen gewonnen werden können. Dieses Prinzip illustriert die Figur 4. Es ist somit erfindungsgemäß bevorzugt, dass die erfindungsgemäßen dreidimensionalen Strukturen oder funktionalisierten Sensorfolien eine Fase an der Öffnung der Kavitäten aufweisen. In Figur 5 werden die Dimensionen der Fase durch einen Durchmesser bzw. (in kanalförmigen Kavitäten) durch eine Breite D2 bzw. B3, jeweils gemessen am obersten Rand der Fase, sowie durch eine Höhe der Fase T2 bzw. T3, jeweils gemessen vom oberen Rand der Kavität bis zum oberen Rand der Fase, illustriert.

Solche Fasen weisen bevorzugt einen Öffnungswinkel von 1 bis 179°, bevorzugt von 40 bis 120°, noch bevorzugter von 60 bis 90° auf. Figur 5A und 5B illustrieren diesen Öffnungswinkel $\alpha$.

Ganz besonders bevorzugte Ausführungsformen der Erfindung umfassen Mikrokavitätenarrays, umfassend 64 Mikrokavitäten in einer 8x8 Anordnung, worin die Mikrokavitäten einen Durchmesser von 300 $\mu$m und eine Gesamttiefe von 250 bis 450 $\mu$m aufweisen und worin eine Fase mit einem Öffnungswinkel von 60 bzw. 90° vorgesehen ist. In solchen Ausführungsformen beträgt der maximale Durchmesser im oberen Bereich der Fase (D2) 500 $\mu$m (vgl. Figur 5).

Neben den vorstehend beschriebenen statischen Systemen, worin die Strukturen als Mikrokavitätenarrays, in Zellkulturinserts oder in Multiwellplatten (z.B. als Boden) eingesetzt werden, können Mikrokavitäten(arrays) auch in fluidische Systeme integriert werden, wie in Figur 6A und 6B dargestellt. Fluidische Systeme können als Mikrobioreaktoren oder Plattformen aus dem Bereich der Organ-on-Chip-Technologien realisiert werden.

In einer weiteren Ausführungsform der Erfindung weisen die dreidimensionalen Strukturen, oder die Mikrokavitätenarrays, Zellkulturinserts oder Multiwellplatten, die aus den bzw. unter Verwendung der erfindungsgemäßen dreidimensionalen Strukturen gebildet werden, einen oder mehrere Bereiche auf, welche eine zusätzliche Funktionalisierung aufweisen, wobei die zusätzlichen funktionalisierten Bereiche bevorzugt in Form von Spots ausgebildet sind, die eine Sauerstoff-, $CO_2$-, Glukose- oder pH-Sensitivität aufweisen, und die räumlich getrennt von den funktionalisierten Mikrokavitäten angeordnet sein können (sogenannte Funktionalisierungs- oder Sensorspots). Solche zusätzlichen Funktionalisierungs-Spots können ebenfalls aus fluoreszierenden Sensorfolien, z.B. Fluorophor-dotierten Sensorfolien, gebildet werden. Es ist dabei möglich, dass die die Mikrokavitäten bildenden funktionalisierten Sensorfolien und die zusätzlichen Sensorspots eine gleiche oder eine unterschiedliche Fluorophor-Dotierung aufweisen, z.B. mit unterschiedlichen Fluorophoren dotiert sind. In einer bevorzugten Ausführungsform ist diese zusätzliche Fluorophor-dotierung der Sensorspots sensitiv gegenüber einem anderen Analyten als die dreidimensionale Struktur aus einer funktionalisierten Sensorfolie der Mikrokavitäten, um so die Messung von mehr als einem Analyten an unterschiedlichen Messpunkten im System in den erfindungsgemäßen Messverfahren zu ermöglichen. In einer besonders bevorzugten Ausführungsform werden die erfindungsgemäßen aus einer funktionalisierten Sensorfolie gebildeten Mikrokavitäten in ein Zellkulturinsert integriert und ein oder mehrere zusätzliche Sensorspots werden räumlich getrennt davon auf dem Insert angebracht. Figur 8 illustriert eine solche beispielhafte Ausführungsform.

Die vorliegende Erfindung umfasst somit auch fluidische Systeme und Mikrobioreaktoren, welche die erfindungsgemäßen dreidimensionalen Strukturen oder funktionalisierten Sensorfolien, sowie ggf. einen oder mehrere der oben beschriebenen Sensorspots oder anderweitige Flurophor-dotierte Bereiche, umfassen. Fluidische Systeme und Mikrobioreaktoren sind grundsätzlich bekannt und können beispielsweise die folgenden Elemente umfassen:

Ein Zellkulturkompartiment, ein Mediumreservoir, eine Pumpe und Ventile, sowie eine Begasungsvorrichtung, wie diese im Stand der Technik zusammengefasst dargestellt sind (z.B. Christoph Grün, Brigitte Altmann, Eric Gottwald: Advanced 3D Cell Culture Techniques in Micro-Bioreactors, Part I: A Systematic Analysis of the Literature Published between 2000 and 2020. Processes 2021, 9:21, DOI: 10.3390/pr8121565; Brigitte Altmann, Christoph Grün, Cordula Nies, Eric Gottwald: Advanced 3D Cell Culture Techniques in Micro-Bioreactors, Part II: Systems and Applications. Processes 2020, 8: 1656, DOI: 10.3390/9010021).

Die vorliegende Erfindung betrifft außerdem Verfahren zur Herstellung Analyt-sensitiver dreidimensionaler Strukturen wie hierin beschrieben. Solche Verfahren umfassen insbesondere die folgenden Schritte:

- Bereitstellung funktionalisierter Sensorfolien, insbesondere solcher wie vorstehend beschrieben insbesondere solcher die mit einem oder mehreren Fluorophoren dotiert sind;
- Verformung der funktionalisierten Sensorfolien;
- und Ausbildung der Analyt-sensitiven dreidimensionalen Strukturen.

Die Verformung der funktionalisierten Sensorfolien erfolgt dabei bevorzugt mittels Mikrothermoformverfahren, insbesondere einem Mikrothermoformverfahren wie als Verfahrensschritt c) in DE10 2004 035 267 B3 beschrieben. Dabei erfolgt ein thermisches Verformen der Sensorfolie zu einem Formkörper. Hierfür kann beispielsweise das allgemein unter dem Namen Mikrothermoformen bekannte Verfahren eingesetzt werden.

Das Mikrothermoformverfahren, wie in DE 10 2004 035 267 B3 beschrieben, umfasst danach insbesondere die Schritte

- thermisches Verformen der Sensorfolie zu den Strukturen unter Verwendung einer Formmaske, wobei die Temperatur unterhalb der Schmelztemperatur des Kunststoffs der Trägerfolie bleibt,
- Entnehmen der so geformten Strukturen aus der Formmaske.

Das erfindungsgemäße Verfahren umfasst insbesondere auch die folgenden weiteren Schritte:

- Herstellung oder Bereitstellung einer Formmaske mit einer Negativform der dreidimensionalen (Mikro)Struktur, umfassend eine oder mehrere Kavitäten sowie optional eine Fase an der Öffnung der Kavitäten;
- Abformen der funktionalisierten Sensorfolien in der Maske;
- Erhalt der Analyt-sensitiven dreidimensionalen Strukturen, insbesondere solcher in Form von (Mikro)Kavitäten, welche gegebenenfalls eine Fase an der Kavitätenöffnung aufweisen.

Bevorzugt werden in dem erfindungsgemäßen Verfahren die Analyt-sensitiven dreidimensionalen Strukturen in Form von Mikrokavitätenarrays, Mikrokanälen oder anderen Mikrovertiefungen für die Anwendung bzw. den Einsatz in Mikrotiterplatten, in Zellkulturplatten oder in Zellkultur-Inserts mit einem oder mehreren Kompartimenten ausgebildet (geformt).

In erfindungsgemäßen Verfahren zur Herstellung von Mikrokavitätenarrays unter Verwendung einer Formmaske wird zunächst die Formmaske für den Mikrothermoformprozess entworfen, welche die Negativform zur gewünschten Zielstruktur darstellt. Für bevorzugte Ausführungsformen mit Fase umfasst die Formmaske an der Öffnung zur Kavität die Fase mit dem gewünschten Öffnungswinkel. Solche Formmasken können beispielsweise aus Messing gefertigt sein. Durch Abformen der Analyt-sensitiven Folien können die gewünschten Strukturen (z.B. Mikrokavitäten) hergestellt werden, insbesondere auch solche, die im Öffnungsbereich über eine Fase zur Bestimmung von Analytgradienten verfügen.

Diese geformten Analyt-sensitiven Folien können dann als solche als Mikrokavitätenarrays eingesetzt oder in geeignete Halterungen wie Zellkulturinserts eingesetzt werden. Desweiteren können Mikrokavitätenarrays auch den Boden von Multiwellplatten selbst darstellen, indem diese mit geeigneten Verfahren an bodenlose Multiwellplatten gebondet werden.

Zur Herstellung der oben beschriebenen Ausführungsformen mit zusätzlichen bzw. unterschiedlich funktionalisierten Bereichen, z.B. in Form von Sensorspots, umfasst das erfindungsgemäße Verfahren außerdem die Aufbringung zusätzlicher funktionalisierter Bereiche, z.B. durch Kombination von gleichen oder unterschiedlichen funktionalisierten Sensorfolien oder geeigneten Beschichtungsmaterialien in unterschiedlichen Bereichen der Mikrokavitäten. Solche unterschiedlichen Funktionalisierungsbereiche, z.B. in Form von Sensorspots, können prinzipiell auf der gesamten Fläche der Mikrokavitäten selbst angeordnet bzw. ausgebildet werden, oder z.B. räumlich getrennt von den die Mikrokavitäten bildenden funktionalisierten Sensorfolien angeordnet werden, z.B. am Rand von Zellkulturinserts.

Die vorliegende Erfindung umfasst außerdem Verfahren zur (non-invasiven) Messung von Analyten oder Analytgradienten in einer Zellkultur unter Verwendung der erfindungsgemäßen dreidimensionalen Strukturen.

Solche Verfahren umfassen insbesondere die folgenden Schritte:

a) Zellkultivierung in den Analyt-sensitiven dreidimensionalen Strukturen wie vorstehend beschrieben;

b) Analytmessung mit geeigneten Detektionsmitteln, z.B. mittels Mikroskopie, beispielsweise Konfokalmikroskopie, insbesondere Fluoreszenzlebenszeit-Mikroskopie;

c) automatisierte Erfassung und Auswertung der Messdaten.

Bevorzugt erfolgt darin die Analytmessung an verschiedenen Messpunkten, d.h. ortsaufgelöst, unter Erfassung des Analytgradienten.

Besonders bevorzugt ist dieses Verfahren zur Bestimmung von Sauerstoff-, $CO_2$- und Glukose-Konzentrationen, sowie von pH-Werten, sowie ganz besonders bevorzugt zur Bestimmung der jeweiligen Gradienten. Über die pH-Wertbestimmung lässt sich beispielsweise auch die sogenannte extracellular acidification rate, ECAR, mit dem erfindungsgemäßen Array bestimmen. Dabei kann zum einen der pH-Wert direkt gemessen werden, wenn eine pH-sensitive Folie verwendet wird. Es ist aber auch möglich, zusätzliche Funktionalisierungsspots auf den erfindungsgemäßen dreidimensionalen Strukturen und funktionalisierten Sensorfolien in Form von Mikroarrays aufzubringen. Solche Funktionalisierungsspots sind Bereiche, die zusätzlich einer Analyt-sensitiven Beschichtung versehen sind, beispielsweise indem sie mit einem weiteren Fluorophor beschichtet sind, bevorzugt neben oder innerhalb der Mikrokavitäten. Diese Funktionalisierungsspots sind gegenüber einem anderen Analyten sensitiv als die die Mikrokavitäten bildenden Sensorfolien. Dadurch kann zum Beispiel zusätzlich zum pH-Wert auch die $CO_2$-Konzentration gemessen werden, um zwischen den Kontributionen der Glykolyse- oder Zellatmungsabhängigen ECAR zu unterscheiden. Analyten die grundsätzlich über zusätzliche Funktionalisierungsspots bestimmt werden können, umfassen prinzipiell alle Analyten die auch in der Analytmessung mit den erfindungsgemäßen Mikrokavitätenarrays selbst bestimmt werden können, wie Sauerstoff-, $CO_2$-, oder Glukose-Konzentrationen und pH-Werte.

Es konnte insbesondere gezeigt werden, dass sich mit dem erfindungsgemäßen Verfahren Sauerstoffkonzentrationen in der direkten Mikroumgebung der Zellen messen lassen.

Diese erfindungsgemäßen Bestimmungsverfahren eignen sich insbesondere für Verfahren, worin die Zellkultur eine dreidimensionale Zellkultur ist und worin in Schritt a) eine dreidimensionale Zellkultivierung erfolgt.

Ein besonderer Vorteil der erfindungsgemäßen Bestimmungsverfahren wie vorstehend beschrieben besteht darin, dass diese Hydrogel-frei und ohne Verwendung von Nanopartikel-gebundenen Fluorophoren durchgeführt werden können. Klarstellend ist dabei anzumerken, dass "Hydrogel-frei" bzw. "ohne Verwendung von Nanopartikel-gebundenen Fluorophoren" nicht solche geformten Sensorfolien ausschließt, worin die Fluorophore über solche Mittel in der Sensorfolie dotiert (gebunden) vorliegen.

Die Erfindung umfasst außerdem die Verwendung der hierin beschriebenen dreidimensionalen Strukturen und funktionalisierten Sensorfolien zur simultanen Zellkultur und Analytmessung, insbesondere zur Messung von $O_2$, $CO_2$, Glukose, pH-Werten und Gradienten davon, wobei die Messung von Sauerstoff und Sauerstoffgradienten ganz besonders bevorzugt ist.

Die Erfindung umfasst außerdem die Verwendung der dreidimensionalen Strukturen oder funktionalisierten Sensorfolien, sowie der fluidischen Systeme oder Mikrobioreaktoren, wie hierin beschrieben zur Anwendung in Hochdurchsatzverfahren. Durch die Möglichkeit der Ausbildung von Arrays mit einer Vielzahl systematisch angeordneter Mikrokavitäten (wie oben beschrieben), eignen sich diese für solche Hochdurchsatzverfahren besonders.

Die Erfindung umfasst außerdem die Verwendung der dreidimensionalen Strukturen oder funktionalisierten Sensorfolien wie hierin beschrieben zur Durchführung von Langzeitstudien, da die Bereitstellung der erfindungsgemäßen Mikrokavitätenarrays eine *in situ*-Zellkultivierung gleicher oder unterschiedlicher Zelltypen simultan und parallel ermöglicht. Es ist möglich, in den erfindungsgemäßen Mikrokavitätenarrays unterschiedliche Kultivierungszeiten anzuwenden. Durch die Möglichkeit des Einsatzes der erfindungsgemäßen Mikrokavitätenarrays in automatisierten Mikroskopplattformen und der Möglichkeit der Zellkultivierung unter physiologischen Bedingungen, eignen sich diese erstmals auch für den Einsatz in Untersuchungen über längere Zeiträume von mehr als 60 min. bis hin zu mehreren Wochen oder Monaten.

Mit den erfindungsgemäßen Mikrokaviätenarrays besteht die Möglichkeit zur Durchführung von Experimenten unter den für die eingesetzte(n) Zelle(n) gewebetypischen Sauerstoffkonzentrationen, da die Mikrokavitätenarrays über die nach oben offenen Kavitäten einer Begasung zugänglich sind. Dadurch wird erstmals die Möglichkeit geschaffen, eine Zellkultivierung unter "physiologischen Bedingungen" durchzuführen, da die Messung von Sauerstoff in Geweben bislang nicht gut funktioniert und/oder nicht durchgeführt werden kann. Durch die erfindungsgemäßen Mikrokavitäten kann ermittelt werden, wie hoch der Sauerstoffgehalt in der Kultur sein muss, damit die eingesetzten Zellen ihre organotypischen Leistungen zeigen.

Grundsätzlich sind die erfindungsgemäßen Systeme zur Kultivierung aller Zellarten / Zelltypen geeignet. Ein besonderer Vorteil der Erfindung liegt jedoch in der besonderen Eignung zur Kultivierung dreidimensionaler Zellkulturen und sphärischer Zellaggregate. Um die Kultivierung von 3D-Zellaggregaten zusätzlich zu fördern, können die Mikrokavitäten aus den funktionalisierten Sensorforlien vor der Zellkultivierung mit Plasma behandelt und/oder mit geeigneten Beschichtungsmitteln behandelt werden, z.B. mit anti-adhäsiven Beschichtungslösungen oder extrazellulärer Matrix (z.B. Collagen). Solche Beschichtungsmittel / Lösungen sind prinzipiell bekannt und kommerziell erhältlich.

Ein weiterer Aspekt der Erfindung betrifft die Verwendung der hierin beschriebenen erfindungsgemäßen dreidimensionalen Strukturen oder funktionalisierten Sensorfolien, sowie fluidischen Systeme und Mikrobioreaktoren in Verfahren zur Charakterisierung von Zellkulturen, von Zellkulturbedingungen, zur Entwicklung von Zell-basierten Gewebemodellen, zur Testung von Wirkstoffen und in Toxizitätsuntersuchungen mittels Zellkulturverfahren, insbesondere zur Verwendung in der Charakterisierung von Zellkulturbedingungen, zur Entwicklung Zell-basierter Modelle für Metabolismusstudien, zur Testung von Wirkstoffen, zur Testung der (ischämischen) Toxikologie *in vitro,* oder zur Ausführung eines Mito-Stress-Tests wie beispielsweise beschrieben in [Christoph Grün, Jana Pfeifer, Gregor Liebsch, Eric Gottwald: O2-sensitive mircocavity arrays: A new platform for oxygen measurements in 3D cell cultures. Frontiers in Bioengineering and Biotechnology 2023, DOI: 10.3389/fbioe.2023.1111316*]*. In einer besonderen Ausführungsform der Erfindung, werden die zuvor genannten Untersuchungsparameter in einem Hochdurchsatzverfahren an vielen Zellkulturen zugleich ausgeführt. Ebenfalls bevorzugt im Sinne der Erfindung ist die Möglichkeit, Langzeitstudien an den kultivierten Zellen vorzunehmen.

Die Erfindung umfasst außerdem Vorrichtungen umfassend

- die erfindungsgemäßen, vorstehend beschriebenen, dreidimensionalen Strukturen oder funktionalisierten Sensorfolien zur Zellkultivierung und Probenaufnahme,
- ggf. einen oder mehrere zusätzliche Funktionalisierungsspots mit weiteren Funktionalisierungen zur Bestimmung unterschiedlicher Parameter,

- mindestens ein Detektionselement zur Erfassung der Messdaten von der funktionalisierten Sensorfolie,
- mindestens ein Element zur automatisierten Erfassung von Messdaten, Datenverarbeitung, Datenauswertung und Datenausgabe.

Detektionselemente zur Erfassung der Messdaten von der Sensorfolie umfassen beispielsweise Mikroskope mit Sensor oder Detektionseinrichtung, wie beispielsweise eine Kamera, sowie insbesondere Konfokalmikroskope, welche erfindungsgemäß bevorzugt sind. In einer solchen erfindungsgemäßen Vorrichtung können die dreidimensionalen Strukturen oder funktionalisierten Sensorfolien insbesondere auch durch eines oder mehrere der vorstehend beschriebenen Merkmale charakterisiert werden.

Die vorliegende Erfindung wird durch die nachfolgenden Beispiele und Figuren näher beschrieben, ohne jedoch hierauf beschränkt zu sein.

**BESCHREIBUNG DER FIGUREN**

Fig. 1　(A) Schematische Darstellung einer zweidimensionalen Zellkultur auf kommerziell erhältlichen planaren Sauerstoff-sensitiven Folien, und

(B) Schematische Darstellung einer dreidimensionalen Zellkultur in erfindungsgemäßen Sauerstoff-sensitiven Mikrokavitätenarrays, zur Bestimmung der Sauerstoffkonzentrationen in unmittelbarer Mikroumgebung der Zellen.

Fig. 2　(A) Schematische Darstellung einer 3D-bedruckten planaren Rampe.

(B) Schematische Darstellung einer experimentellen Anordnung mit einer 3D-bedruckten planaren Rampe in einer zweidimensionalen Zellkultur.

Fig. 3　(A) Schematische Darstellung von Mikrostrukturen gemäß Stand der Technik unter Verwendung einer mit einer planaren Sensorfolie bedruckten Rampe als Insert auf dem Boden einer 2D-Zellkultur zur Messung von Sauerstoffgradienten

(B) Schematische Darstellung von Mikrostrukturen gemäß Stand der Technik unter Verwendung einer mit einer planaren Sensorfolie bedruckten Rampe als Insert eingehängt in eine 2D-Zellkultur zur Messung von Sauerstoffgradienten.

(C) Schematische Darstellung von Mikrostrukturen gemäß Stand der Technik unter Verwendung einer mit einer planaren Sensorfolie bedruckten Rampe als Insert eingehängt in eine 3D-Zellkultur zur Messung von Sauerstoffgradienten.

(D) Schematische Darstellung von erfindungsgemäßen Mikrokavitäten mit Fase am Boden eines Inserts zur Messung von Sauerstoffgradienten in kugelförmigen 3D-Zellkulturen.

(E) Schematische Darstellung von erfindungsgemäßen Mikrokavitäten mit Fase am Boden eines Wells zur Messung von Sauerstoffgradienten in kugelförmigen 3D-Zellkulturen.

(F) Detaildarstellung des Zellaggregats in der Mikrokavität aus einer Sensorfolie und Möglichkeiten der Sauerstoffbestimmung.

Fig. 4 (A) Mikrokavitätenarray mit Fase.
(B) Schematische Darstellung einer erfindungsgemäßen Mikrokavität aus funktionalisierter Sensorfolie, erhalten mittels Thermoformverfahren, und kugelförmigen 3D-Zellaggregaten, sowie deren Messung mittels Mikroskop.

Fig. 5 (A) Schematische Darstellung einer erfindungsgemäßen runden Kavität mit Fase im Querschnitt.

(B) Schematische Darstellung einer erfindungsgemäßen kanalförmigen Kavität mit Fase im Querschnitt.

(C) Schematische Darstellung einer erfindungsgemäßen runden Kavität mit Fase in der Aufsicht.

(D) Schematische Darstellung einer erfindungsgemäßen kanalförmigen Kavität mit Fase in der Aufsicht.

Fig. 6 (A) Fluidische Systeme / Zellkultur-Inserts & Multiwellplatten.

(B) Fluidische Systeme / Mikrobioreaktoren.

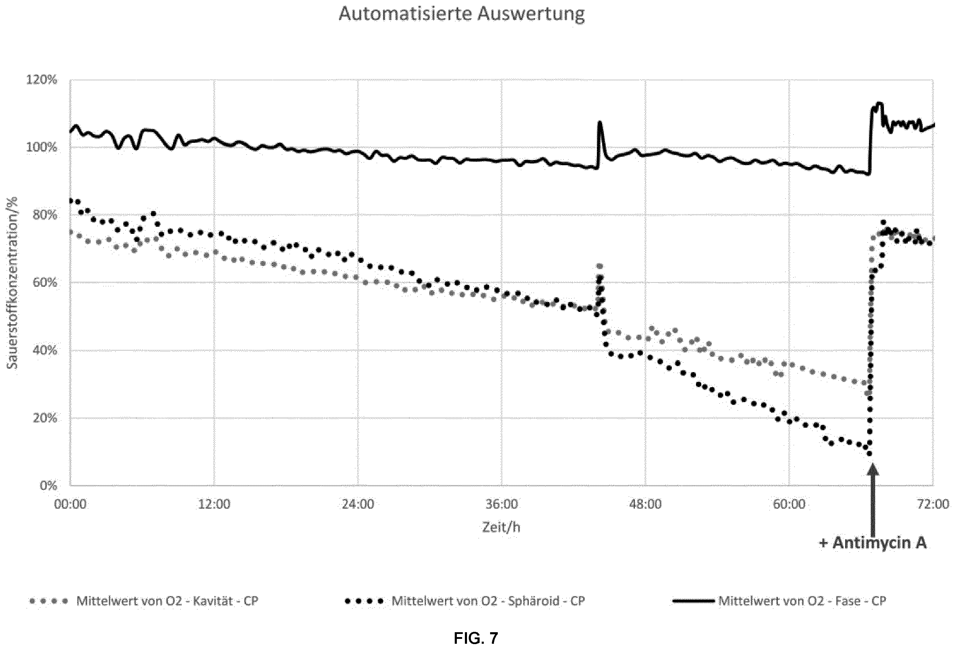

Fig. 7 Sauerstoff-Messung mit automatisierter Auswertung

Fig. 8 Zellkulturinsert umfassend erfindungsgemäße Mikrokavitäten und zusätzliche Sensorspots für die Messung weiterer Analyten.

Fig. 9 Mito-Stresstest ausgeführt in den erfindungsgemäßen Mikrokavitäten mit Fase an HepG2-Sphäroiden. Die Sauerstoffkonzentration in Abhängigkeit von der Zeit an drei Bereichen in Sphäroidumgebung wird dargestellt: 0 - 30 min Gleichgewichtseinstellung; 30 - 75 min Kontrollzugabe von Kulturmedium mit HEPES, Bestimmung basaler Zellatmung; 75 - 120 min: Zugabe von 4 μM Oligomycin, Bestimmung ATP-abhängiger Zellatmung; 120 - 165 min: Zugabe von 2 μM Carbonylcyanid-4-(trifluormethoxy)phenylhydrazon (FCCP), Bestimmung der maximalen Zellatmung; 165 - 210 min: Zugabe von 1 M Rotenon und 1μM Antimycrin A, Bestimmung der nicht-mitochondrialen Zellatmung.

**BEZUGSZEICHEN**

(1) Dreidimensionale Struktur(en) (Mikrostrukturen / Mikrokavität(en))
(2) Mikrokavitätenarrays / Mikrotiterplatten / Wellplatten / Well
(3) Insert
(4) funktionalisierte Sensorfolie
(5) Fase(n)
(6) Zellen / (3D-)Zellkultur / Sphäroid(e)
(7) Detektionselement / Mikroskop
(8) Rampe
**(9)** Funktionalisierungsspot / Sensorspot

BEISPIELE

1. Ausführungsbeispiele von erfindungsgemäßen dreidimensionalen Strukturen

Mögliche Ausführungsbeispiele sind in den folgenden Figuren dargestellt:

**Figur 3D:**

Erfindungsgemäße Mikrokavitäten mit Fase am Boden eines Inserts zur Messung von Analytgradienten in kugelförmigen 3D-Zellkulturen.

**Figur 3E:**

Erfindungsgemäße Mikrokavitäten mit Fase am Boden eines Wells zur Messung von Analytgradienten in kugelförmigen 3D-Zellkulturen.

**Figur 3F:**

(Halb)runde Mikrokavität aus einer Sensorfolie mit 3D-Zellaggregat.

**Figur 4A:**

Mikrokavitätenarray mit (halb)runden Mikrokavitäten, jeweils mit Fase.

**Figur 5A und 5C:**

(Halb)runde Mikrokavität mit Fase.

**Figur 5B und 5D:**

Kanalförmige Kavität mit Fase.

2. Ausführungsbeispiele erfindungsgemäßer fluidischer Systeme und Zellkulturinserts

**Figur 6A:**

Zellkultur-Inserts & Multiwellplatten umfassend dreidimensionale Mikrostrukturen

**Figur 6B:**

Mikrobioreaktoren umfassend dreidimensionale Mikrostrukturen

**Figur 8:**

Zellkulturinsert umfassend dreidimensionale Mikrostrukturen und zusätzliche Sensorspots

3. Herstellungsbeispiel erfindungsgemäßer dreidimensionaler Mikrostrukturen

Es wurden Mikrokavitätenarrays hergestellt, indem zunächst eine Formmaske (Negativ-Maske) aus Messing gefertigt wurde, welche an der Öffnung zu den Kavitäten jeweils eine Fase umfassen.

In der Formmaske wurde eine für das Mikrothermoformen optimierte Sauerstoff-sensitive Folie der Firma PreSens Precision Sensing GmbH mittels Mikrothermoformverfahren gemäß DE10 2004 035 267 B3 unter Erhalt der Mikrokavitäten, die im Öffnungsbereich über eine Fase zur Bestimmung von Gradienten verfügen, abgeformt.

4. Anwendungsbeispiel erfindungsgemäßer dreidimensionaler Mikrostrukturen

In den gemäß Beispiel 3 hergestellten Mikrokavitätenarrays wurden HepG2-Zellen als kugelige Aggregate (Sphäroide) kultiviert und die Sauerstoffkonzentration im Bereich der Fase mittels Mikroskopie mit einem VisiSens-System der Firma PreSens Precision Sensing GmbH gemessen.

Figur 7 zeigt das Ergebnis der gemittelten Messergebnisse im Bereich der gesamten Mikrokavität, im Bereich des Sphäroids (im Zentrum der Mikrokavität) sowie im Bereich der Fase. Nach 80 h wurde 1 μM Antimycin A, ein Inhibitor der Zellatmung, hinzugegeben. Dies führte zu einem Anstieg der Sauerstoffkonzentration, da mehr Sauerstoff in die Kavität diffundierte als von den Zellen verbraucht wurde.

Die Auswertung der Sauerstoffkonzentrationen (Figur 7) zeigt den Unterschied der Konzentration zwischen dem Bereich der Fase und den Bereichen der eigentlichen Kavität bzw. der Sphäroide. Die Messung des Sauerstoffgradienten kann durch Anpassung in der Auswertesoftware durch eine andere Segmentierung der zu messenden Flächen detaillierter erfolgen.

Für die Bestimmung wurde eine Fluorophormischung aus einem Sauerstoff-sensitiven Fluorophor und einem Referenzfluorophor verwendet, um die Daten mit dem VisiSens-System auswerten zu können.

Das Verfahren ist analog mit anderen funktionalisierten Sensorfolien unter Verwendung anderer Dotierungen (bspw. anderer Fluorophore, die auf die zu messenden Analyten sensitiv sind) auch zur Messung anderer Analyten einsetzbar, worin die Auswertung der Signale über Fluoreszenzlebensdauer- bzw. Phosphoreszenzlebensdauer-Mikroskopie erfolgen kann.

Mit diesem Verfahren sind Messungen mit hoher z-Auflösung möglich.

5. Durchführung eines Mito-Stresstest

Die hierin, insbesondere in den Beispielen 1 bis 3, beschriebenen Mikrokavitätenassays auf Basis Sauerstoff-sensitiver Folien wurden für die Testung von Wirkstoffen zur Untersuchung der ischämischen Toxikologie *in vitro* wie im Detail in [Christoph Grün, Jana Pfeifer, Gregor Liebsch, Eric Gottwald: O2-sensitive mircocavity arrays: A new platform for oxygen measurements in 3d cell cultures. Frontiers in Bioengineering and Biotechnology 2023, DOI: 10.3389/fbioe.2023. 1111316*]* beschrieben, eingesetzt.

Es wurden HepG2-Zellen unter Standardbedingungen in einer feuchten Atmosphäre mit 5 % $CO_2$- und 18,6 % $O_2$-Gehalt bei 37°C kultiviert.

Mikrokavitätenarrays die gemäß Beispiel 3 hergestellt wurden, wurden mit einem Film aus BIOFLOAT™ der Firma FaCellitate beschichtet. In den beschichteten Kavitäten wurden kugelige Aggregate (Sphäroide) der kultivierten HepG2 Zellen gebildet.

Die so kultivierten Sphäroide in den erfindungsgemäßen Mikrokavitätenarrays wurden anschließend mittels Mikroskopie mit dem VisiSens-System der Firma PreSens Sensing GmbH hinsichtlich der Sauerstoffkonzentration in der Sphäroidumgebung untersucht.

Es wurde eine Kalibrierung zur Bestimmung einer maximalen und minimalen Sauerstoffkonzentration in den Kavitäten auf den erfindungsgemäßen Mikrokavitätenarrays ausgeführt. Dazu wurde zum einen die Sauerstoffkonzentration in einem zuvor 10 min unter den Kultivierungsbedingungen equilibrierten 100 μL Wassertropfen gemessen, um die maximale Sauerstoffkonzentration zu bestimmen. Zum anderen wurde dieser Vorgang mit einer 10 mg/ml Natriumsulfit Lösung zur Bestimmung der minimalen Sauerstoffkonzentration wiederholt.

Im Anschluss daran wurde mit den erfindungsgemäßen Mikrokavitätenarrays die Sauerstoffkonzentration an drei ausgewählten Positionen in Sphäroidumgebung unter verschiedenen Bedingungen untersucht. Messungen wurden in 2-minütigen Intervallen ausgeführt. Dadurch wird ein Aufschluss über das Zellatmungsverhalten unter den untersuchten Gegebenheiten erlangt.

Die Bestimmung der Sauerstoffkonzentration in der Sphäroidumgebung erfolgte an Sphäroiden in den erfindungsgemäßen Mikrokavitätenarrays im Kulturmedium, welches mit 10mM HEPES versetzt wurde.

Die Sauerstoffkonzentration an den ausgewählten Stellen der Sphäroidumgebung wurde in einem ersten Schritt für 30 min gemessen, um die Einstellung eines Gleichgewichtes in der Sauerstoffkonzentration zu erfassen.

In einem zweiten Schritt wurde nach 30 min erneut Kulturmedium, welches mit HEPES versetzt wurde, zugesetzt und die Sauerstoffkonzentration weiter gemessen, um die basale Zellatmung zu bestimmen.

In einem dritten Schritt wurden nach weiteren 30 min 4 $\mu$M Oligomycin zugesetzt und die Sauerstoffkonzentration weiter gemessen, um die ATP-abhängige Zellatmung zu erfassen.

In einem vierten Schritt wurden nach weiteren 30 min 2 $\mu$M Carbonylcyanid-4-(trifluormethoxy)phenylhydrazon (FCCP) zugesetzt und die Sauerstoffkonzentration weiter gemessen, um die maximale Zellatmung zu erfassen.

In einem fünften Schritt wurden nach weiteren 30 min je 1 $\mu$M Rotenon und 1 $\mu$M Antimycin A zugesetzt und die Sauerstoffkonzentration weiter gemessen, um die nicht-mitochondriale Zellatmung zu erfassen.

Die Sauerstoffkonzentration in Abhängigkeit von der Zeit und Zugabe der diversen Testsubstanzen ist in Figur 9 dargestellt. Die Figur zeigt, wie die Sauerstoffkonzentration im Kulturmedium an drei verschiedenen Stellen in der Sphäroidumgebung nach Zugabe der verschiedenen Testsubstanzen steigt oder fällt. Eine steigende Sauerstoffkonzentration spricht für eine inhibierte Zellatmung durch die Testsubstanz, da weniger Sauerstoff aus der Umgebung durch die Sphäroide verbraucht wird. Die drei unterschiedlichen Messkurven für die unterschiedlichen Positionen in der Sphäroidumgebung verdeutlichen die dreidimensionale Ortsabhängigkeit des Sauerstoffverbrauchs um die Sphäroide.

## Patentansprüche

1. Dreidimensionale Strukturen, die aus Fluorophordotierten, funktionalisierten Sensorfolien gebildet sind.

2. Funktionalisierte Sensorfolien, welche Fluorophordotierte Folien sind, in Form von dreidimensionalen Strukturen zur Aufnahme und/oder Züchtung von Zellen.

3. Dreidimensionale Strukturen nach Anspruch [1] oder funktionalisierte Sensorfolien nach Anspruch [2], wobei es sich bei den Strukturen um Mikrokavitäten handelt.

4. Dreidimensionale Strukturen oder funktionalisierte Sensorfolien nach Anspruch [3], wobei es sich bei den Strukturen um Mikrokavitäten handelt, welche in Form von Mikrokavitätenarrays, Mikrokanälen oder anderen Mikrovertiefungen für die Anwendung (den Einsatz) in Mikrotiterplatten, in Zellkulturplatten oder in Zellkultur-Inserts mit einem oder mehreren Kompartimenten vorliegen.

5. Dreidimensionale Strukturen oder funktionalisierte Sensorfolien nach einem der Ansprüche [1] bis [4], worin die Fluorophor-dotierten Sensorfolien Sauerstoff-, $CO_2$-, Glukose- oder pH-sensitive Fluorophore umfassen.

6. Dreidimensionale Strukturen oder funktionalisierte Sensorfolien nach Anspruch [5] worin die funktionalisierten Sensorfolien aus Sauerstoff-sensitiven Folien ausgewählt sind.

7. Dreidimensionale Strukturen oder funktionalisierte Sensorfolien nach einem der Ansprüche [1] bis [6], worin die Kavitäten eine Fase an der Öffnung der Kavitäten aufweisen.

8. Dreidimensionale Strukturen oder funktionalisierte Sensorfolien nach Anspruch [7], worin die Fasen an den Öffnungen der Mikrokavitäten einen Öffnungswinkel von 1 bis 179° aufweisen.

9. Dreidimensionale Strukturen oder funktionalisierte Sensorfolien nach einem der Ansprüche [1] bis [8], die zusätzlich einen oder mehrere Bereiche aufweisen, welche bevorzugt in Form von Spots ausgebildet sind, die eine Sauerstoff-, $CO_2$-, Glukose- oder pH-Sensitivität aufweisen und an einem oder mehreren beliebigen Punkten oder Bereichen auf der Oberfläche der dreidimensionalen Strukturen oder funktionalisierten Sensorfolien oder räumlich getrennt von den funktionalisierten Mikrokavitäten angeordnet sind.

10. Fluidische Systeme oder Mikrobioreaktoren umfassend die dreidimensionalen kavitätischen Strukturen oder funktionalisierten Sensorfolien nach einem der Ansprüche [1] bis [9].

11. Verfahren zur Herstellung Analyt-sensitiver dreidimensionaler Strukturen wie in einem der Ansprüche [1] bis [9] definiert, umfassend die Schritte

• Bereitstellung funktionalisierter Sensorfolien, welche mit einem oder mehreren Fluorophoren dotiert sind;
• Verformung der funktionalisierten Sensorfolien mittels Mikrothermoformverfahren;
• und Ausbildung der Analyt-sensitiven dreidimensionalen Strukturen, insbesondere in Form von Mikrokavitätenarrays, Mikrokanälen oder anderen Mikrovertiefungen für die Anwendung (den Einsatz) in Mikrotiterplatten, in Zellkulturplatten oder in Zellkultur-Inserts mit einem oder mehreren Kompartimenten.

12. Verfahren gemäß Anspruch [11], umfassend außerdem die

• Aufbringung zusätzlicher funktionalisierter Bereiche, vorzugsweise in Form von Sensorspots, und/oder
• Behandlung der die Mikrokavitäten bildenden funktionalisierten Sensorfolien mit Plasma und/oder Beschichtung mit Beschichtungsmitteln, wie anti-adhäsiven Mitteln oder extrazellulärer Matrix (z.B. Collagen).

13. Verfahren zur (non-invasiven) Messung von Analyten oder Analytgradienten in einer Zellkultur, umfassend

a) Zellkultivierung, insbesondere dreidimensionale Zellkultivierung, in den Analyt-sensitiven dreidimensionalen Strukturen wie in [1] bis [9] definiert;
b) Analytmessung mittels Mikroskopie, insbesondere an verschiedenen Messpunkten unter Erfassung eines Analyt-Gradienten, insbesondere zur Bestimmung von Sauerstoff-, $CO_2$-, Glukosekonzentrationen oder pH-Werten oder deren Gradienten;
c) automatisierte Erfassung und Auswertung der Messdaten.

14. Verwendung der dreidimensionalen Strukturen oder funktionalisierten Sensorfolien nach einem der Ansprüche [1] bis [9] zur simultanen Zellkultur und Analytmessung von einem oder mehreren Analyten, insbesondere zur Messung von $O_2$, $CO_2$, Glukose, pH-Werten und Gradienten davon.

(A)

(B)

**FIG. 1**

(A)

(B)

**FIG. 2**

FIG. 3

(A)

(B)

FIG. 4

(A)

(B)

(C)

(D)

FIG. 5

(A)

(B)

FIG. 6

FIG. 8

FIG. 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102007007718 A1
- DE 102009044115 A1
- DE 102004035267 B3
- WO 201908461 A1

**In der Beschreibung aufgeführte Nicht-Patentliteratur**

- **WANG, X.D. ; O.S. WOLFBEIS.** Optical methods for sensing and imaging oxygen: materials, spectroscopies and applications. *Chem Soc Rev,* 2014, vol. 43 (10), 3666-761
- **NICHOLS, A.J. et al.** Click-assembled, oxygen-sensing nanoconjugates for depth-resolved, near-infrared imaging in a 3D cancer model. *Angew Chem Int Ed Engl,* 2014, vol. 53 (14), 3671-4
- **EGGERT, S. et al.** Automated 3D Microphysiometry Facilitates High-Content and Highly Reproducible Oxygen Measurements within 3D Cell Culture Models. *ACS Sens,* 2021, vol. 6 (3), 1248-1260
- **BABILAS, P. et al** *In vivo phosphorescence imaging of pO2 using planar oxygen sensors: Microcirculation,* 2005, vol. 12 (6), 477-87
- **KELLNER, K. et al.** Determination of oxygen gradients in engineered tissue using a fluorescent sensor. *Biotechnol Bioeng,* 2002, vol. 80 (1), 73-83
- **VALIMAKI, H. et al.** Covalent immobilization of luminescent oxygen indicators reduces cytotoxicity. *Biomed Microdevices,* 2020, vol. 22 (2), 41
- **SCHREML et al.** Luminescent Dual Sensors Reveal Extracellular pH-Gradients and Hypoxia on Chronic Wounds That Disrupt Epidermal Repair. *Theranostics,* 2014, vol. 4 (7), 721-735
- **VON PENICHE SILVA et al.** A New Non-invasive Technique for Measuring 3D-Oxygen Gradients in Wells During Mammalian Cell Culture. *Original Research,* 17. Juni 2020
- **CHRISTOPH GRÜN ; BRIGITTE ALTMANN ; ERIC GOTTWALD.** Advanced 3D Cell Culture Techniques in Micro-Bioreactors, Part I: A Systematic Analysis of the Literature Published between 2000 and 2020. *Processes,* 2021, vol. 9, 21
- **BRIGITTE ALTMANN ; CHRISTOPH GRÜN ; CORDULA NIES ; ERIC GOTTWALD.** Advanced 3D Cell Culture Techniques in Micro-Bioreactors, Part II: Systems and Applications. *Processes,* 2020, vol. 8, 1656
- **CHRISTOPH GRÜN ; JANA PFEIFER ; GREGOR LIEBSCH ; ERIC GOTTWALD.** O2-sensitive mircocavity arrays: A new platform for oxygen measurements in 3D cell cultures. *Frontiers in Bioengineering and Biotechnology,* 2023
- **CHRISTOPH GRÜN ; JANA PFEIFER ; GREGOR LIEBSCH ; ERIC GOTTWALD.** O2-sensitive mircocavity arrays: A new platform for oxygen measurements in 3d cell cultures. *Frontiers in Bioengineering and Biotechnology,* 2023